# EUROPEAN PATENT APPLICATION

(11) **EP 4 447 061 A1**
(43) Date of publication of application: **16.10.2024**
(21) Application number: 24169301.9
(22) Date of filing: 09.04.2024
(51) Int. Cl.: G16H 10/60, G16H 40/20, G16H 50/20

(54) **ABNORMALITY DETERMINATION SYSTEM AND BASE STATION DEVICE**

(30) Priority: 14.04.2023 JP 2023066753
(71) Applicant: Suzuki Motor Corporation, Shizuoka 432-8611 (JP)
(72) Inventor: TAKAGAITO, Fumiya, Shizuoka, 432-8611 (JP); KAMIYA, Naoki, Shizuoka, 432-8611 (JP); NAGAE, Yusuke, Shizuoka, 432-8611 (JP)
(74) Representative: Haseltine Lake Kempner LLP

(57) **Abstract**

A mobility server that stores user data D2, and a medical server that stores vehicle data D1. The abnormal indication of the user is determined from the vehicle data D1, and medical information created based on information related to the determined abnormal indication of the user and an opinion of a medical worker at the medical institution is notified to a user terminal.

## Description

### TECHNICAL FIELD

The present invention relates to an abnormality determination system and a base station device.

### BACKGROUND ART

For example, Patent Literature 1 discloses a dementia risk determination system in which a situation determination unit determines a driving situation of a vehicle based on road information acquired by a road information acquisition unit and traveling information of the vehicle acquired by a vehicle information acquisition unit, and a violation determination unit determines whether the driving situation corresponds to a predetermined traffic violation that is likely to be performed when a cognitive function deteriorates. In the dementia risk determination system, when the driving situation of the vehicle corresponds to the predetermined traffic violation, a risk determination unit determines whether there is a dementia risk based on information related to a driver of the vehicle and a violation history obtained by a driver information detection unit. When the risk determination unit determines that there is a dementia risk, an output unit outputs the information.

### CITATION LIST

### PATENT LITERATURE

Patent Literature 1: JP2019-124975A

### SUMMARY OF INVENTION

### TECHNICAL PROBLEM

A cognitive function inspection is currently conducted at various facilities to confirm a cognitive function state of an elderly person whose risk of deterioration in the cognitive function increases with age. For example, when renewing a driver's license for the elderly person, the cognitive function inspection is conducted to measure memory and judgment using an inspection form, an electronic terminal, and the like. Such a cognitive function inspection is not equivalent to a medical inspection conducted by a medical institution, but is merely a simple inspection to confirm a state of memory and judgment of an examinee. Although these cognitive function inspections can confirm the cognitive function state at the time of the inspection, the cognitive function inspections cannot confirm the state of the cognitive function in daily life, such as when actually driving. Therefore, it is difficult to detect an abnormality of the cognitive function in an initial stage in which an indication or a symptom of deterioration in cognitive function which the user is not aware of is not always observed. From such a viewpoint, it is desired to discover an indication or a symptom of a disease related to a cognitive function of a user at an early stage from daily behavior such as driving of a vehicle.

The present invention has been made in view of the above circumstances, and an object thereof is to provide a base station device and an abnormality determination system capable of quickly discovering an indication or a symptom of a disease related to a cognitive function of a user by providing, to a medical institution, information related to an abnormal indication of the user in a vehicle.

### SOLUTION TO PROBLEM

In order to achieve the above object, an abnormality determination system according to the present invention includes: a mobility server configured to store user data related to a user of a vehicle; a medical server configured to store vehicle data including traveling information of the vehicle; a determination and analysis device configured to determine an abnormal indication of the user from the vehicle data, store determination result data including a determination result in the medical server, and issue a notification of medical information to the user based on the determination result data; and a user terminal configured to receive the notification from the determination and analysis device. The mobility server and the medical server cooperate with each other to manage the user data, the vehicle data, and the determination result data in association with each other, and the determination and analysis device transmits at least the determination result data to a medical institution terminal of a medical institution, receives medical information data created based on the determination result data and an opinion of a medical worker at the medical institution from the medical institution terminal, and notifies the user terminal of the medical information including a content based on the medical information data.

In order to achieve the above object, a base station device according to the present invention includes: a mobility server configured to store user data related to a user of a vehicle; a medical server configured to store vehicle data including traveling information of the vehicle; and a determination and analysis device configured to determine an abnormal indication of the user from the vehicle data, store determination result data including a determination result in the medical server, and issue a notification of medical information to the user based on the determination result data. The mobility server and the medical server cooperate with each other to manage the user data, the vehicle data, and the determination result data in association with each other, and the determination and analysis device transmits at least the determination result data to a medical institution terminal of a medical institution, receives medical information data created based on the determination result data and an opinion of a medical worker at the medical institution from the medical institution terminal, and notifies a user terminal of the medical information including a content based on the medical information data.

### ADVANTAGEOUS EFFECTS OF INVENTION

An abnormality determination system and a base station device according to the present invention provide information related to an abnormal indication of a user in a vehicle to a medical institution, thereby exerting an effect of being able to discover an indication or a symptom of a disease related to a cognitive function of the user at an early stage.

### BRIEF DESCRIPTION OF DRAWINGS

[FIG. 1] FIG. 1 is a block diagram showing a schematic configuration of an abnormality determination system according to a first embodiment.
[FIG. 2] FIG. 2 is a block diagram showing a schematic configuration of the abnormality determination system according to the first embodiment.
[FIG. 3] FIG. 3 is a schematic diagram illustrating main data treated by the abnormality determination system according to the first embodiment.
[FIG. 4] FIG. 4 is a block diagram showing a schematic configuration of an in-vehicle device included in the abnormality determination system according to the first embodiment.
[FIG. 5] FIG. 5 is a block diagram showing a schematic configuration of a data processing device included in the abnormality determination system according to the first embodiment.
[FIG. 6] FIG. 6 is a block diagram showing a schematic configuration of a mobility server included in the abnormality determination system according to the first embodiment.
[FIG. 7] FIG. 7 is a block diagram showing a schematic configuration of an analysis device included in the abnormality determination system according to the first embodiment.
[FIG. 8] FIG. 8 is a block diagram showing a schematic configuration of a medical server included in the abnormality determination system according to the first embodiment.
[FIG. 9] FIG. 9 is a block diagram showing a schematic configuration of a determination device included in the abnormality determination system according to the first embodiment.
[FIG. 10] FIG. 10 is a schematic diagram showing an example of a database stored in the mobility server and the medical server included in the abnormality determination system according to the first embodiment.
[FIG. 11] FIG. 11 is a block diagram showing a schematic configuration of a user terminal included in the abnormality determination system according to the first embodiment.
[FIG. 12] FIG. 12 is a diagram showing an example of a notification of a consultation recommendation displayed on the user terminal included in the abnormality determination system according to the embodiment.
[FIG. 13] FIG. 13 is a diagram showing an example of the notification of the consultation recommendation displayed on the user terminal included in the abnormality determination system according to the embodiment.
[FIG. 14] FIG. 14 is a diagram showing an example of the notification of the consultation recommendation displayed on the user terminal included in the abnormality determination system according to the embodiment.
[FIG. 15] FIG. 15 is a block diagram showing a schematic configuration of a medical institution terminal included in the abnormality determination system according to the first embodiment.
[FIG. 16] FIG. 16 is a diagram showing an example of a flow (sequence) of a process performed by each processing unit of the abnormality determination system according to the first embodiment.
[FIG. 17] FIG. 17 is a diagram showing an example of a flow (sequence) of a process performed by each processing unit of the abnormality determination system according to the first embodiment.
[FIG. 18] FIG. 18 is a diagram showing an example of a flow (sequence) of a process performed by each processing unit of the abnormality determination system according to the first embodiment.
[FIG. 19] FIG. 19 is a block diagram showing a schematic configuration of an abnormality determination system according to a second embodiment.
[FIG. 20] FIG. 20 is a diagram showing an example of a flow (sequence) of a process performed by each processing unit of the abnormality determination system according to the second embodiment.
[FIG. 21] FIG. 21 is a diagram showing an example of a flow (sequence) of a process performed by each processing unit of the abnormality determination system according to the second embodiment.
[FIG. 22] FIG. 22 is a diagram showing an example of a flow (sequence) of a process performed by each processing unit of the abnormality determination system according to the second embodiment.

### DESCRIPTION OF EMBODIMENTS

Hereinafter, embodiments according to the present invention will be described in detail with reference to the drawings. The present invention is not limited to these embodiments. In addition, constituent elements in the following embodiments include one easily replaced by a person skilled in the art or essentially the same constituent element.

### [First Embodiment]

### <Overview of Abnormality Determination System>

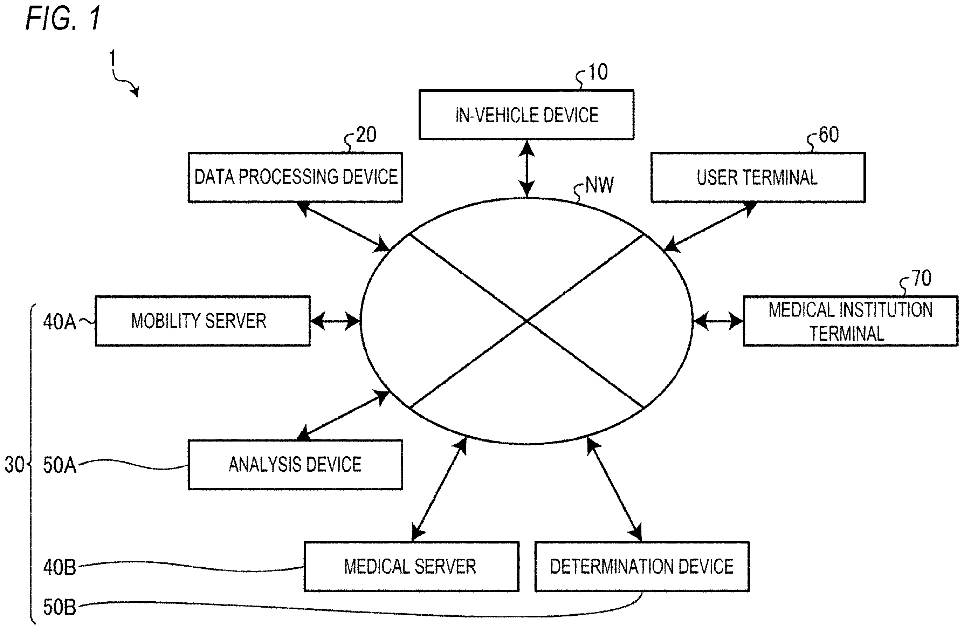

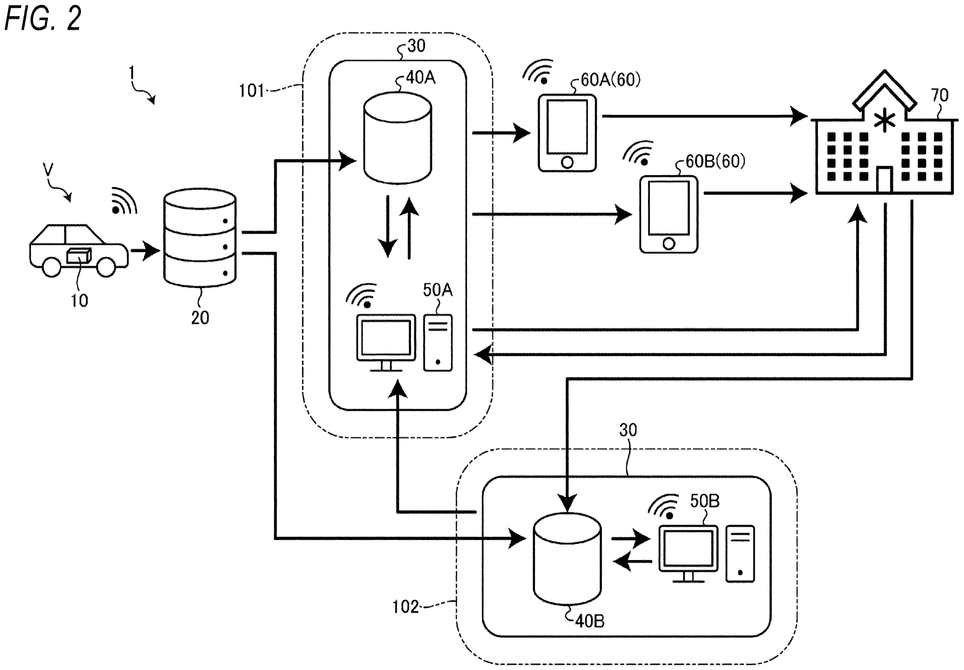

An abnormality determination system 1 according to a first embodiment shown in FIGS. 1 and 2 includes an in-vehicle device 10, a data processing device 20, a service providing device 30, a user terminal 60, and a medical institution terminal 70, and these devices and terminals are configured to communicate with each other through a network NW. The network NW constitutes a communication network that connects a plurality of devices so as to be able to communicate with each other, and enables data communication by various communication methods that can be applied to the present system regardless of wireless communication or wired communication. The network NW may include, for example, an Internet line network, a mobile phone line network, a mobile communication network, a local area network (LAN), a wide area network (WAN), and a virtual private network (VPN), and various network relay devices such as an antenna, a gateway, a router, and a hub may be interposed. The abnormality determination system 1 according to the present embodiment constitutes a system in which the in-vehicle device 10, the data processing device 20, the service providing device 30, the user terminal 60, and the medical institution terminal 70 can perform data communication with each other via the network NW to cooperate with each other to manage various kinds of data related to a vehicle V, and the data can be used for various services and analyses.

In such an abnormality determination system 1, the service providing device 30 includes a mobility server 40A, a medical server 40B, and a determination and analysis device (analysis device 50A and determination device 50B), and various kinds of data treated by the abnormality determination system 1 are stored separately in the mobility server 40A and the medical server 40B. Accordingly, independence of the data stored in the mobility server 40A and the medical server 40B can be increased, and confidentiality of information can be protected. In the present embodiment, the determination and analysis device includes the analysis device 50A and the determination device 50B. The abnormality determination system 1 according to the present embodiment determines presence or absence and a content of an abnormal indication of a user from a daily driving behavior of the user of the vehicle V, and if it is determined that there is an abnormal indication in the user, the abnormality determination system 1 provides determination result data D5 including information related to a determination result to a medical institution. In the abnormality determination system 1 according to the present embodiment, the service providing device 30 receives medical information data D6 created based on the determination result data D5 provided to the medical institution and an opinion of a medical worker such as a doctor in the medical institution, and a notification of medical information including a content based on the medical information data D6 is issued to the user.

Here, the abnormal indication of the user who drives the vehicle V refers to an indication of deterioration in cognitive function of the user. From researches related to a relation between a cognitive function of a driver and a driving behavior, the present inventor has found that there is a correlation relation between and a cognitive function state of the driver and specific driving behaviors, such as sudden braking, sudden steering, and overlooking of a temporary stop sign. Specifically, the present inventor has found that, compared to a case where a person having a normal cognitive function drives the vehicle V, in a case where a person having an abnormal cognitive function drives the vehicle V, sudden braking, sudden steering, and overlooking of a temporary stop sign tend to increase. The present inventor has found that, compared to the case where the person having the normal cognitive function drives the vehicle V, in the case where the person having the abnormal cognitive function drives the vehicle V, an inter-vehicle distance between the vehicle V of the user and a vehicle traveling in front of the vehicle V of the user tends to be shorter than an appropriate inter-vehicle distance. The present inventor considers that the number of times of specific driving behaviors, such as sudden braking, sudden steering, and overlooking of a temporary stop sign, within a predetermined period (for example, within a period where the vehicle V travels 100 km) and the inter-vehicle distance to a preceding vehicle are utilized as one of determination materials for determining presence or absence of an indication or a symptom of a disease related to the cognitive function of the user in the vehicle V In the abnormality determination system 1 according to the present embodiment, the medical worker such as a doctor in the medical institution determines the presence or absence of the indication or the symptom of the disease related to the cognitive function of the user based on the determination result data D5 and his or her own opinion, and information related to the determination result, a consultation recommendation to the medical institution, a treatment program, and the like can be provided.

The abnormality determination system 1 detects the driving behaviors, such as sudden braking, sudden steering, and overlooking of a temporary stop sign, as abnormal driving of the user of the vehicle V The abnormality determination system 1 according to the present embodiment may detect, as the abnormal driving of the user, a matter that sudden braking, sudden steering, overlooking of a temporary stop sign, and the like occur a predetermined number of times or more within the predetermined period (for example, within the period where the vehicle travels 100 km), for example. The abnormality determination system 1 according to the present embodiment determines the presence or absence of the indication (abnormal indication) of deterioration in cognitive function of the user who drives the vehicle V from vehicle data D1 based on a reference created on the basis of a correlation relation between a driving operation of the vehicle V and the cognitive function state of the user, for example, a reference such as whether sudden braking, sudden steering, overlooking of a temporary stop sign, and the like occur a predetermined number of times or more within the predetermined period (for example, within the period where the vehicle V travels 100 km).

In the abnormality determination system 1 according to the present embodiment, the data stored in the mobility server 40A and the medical server 40B is utilized by the determination and analysis device (analysis device 50A and determination device 50B). The determination device 50B in the determination and analysis device determines the presence or absence, the content, and the like of the abnormal indication of each user based on the data stored in the mobility server 40A and the medical server 40B. Then, the analysis device 50A in the determination and analysis device provides, to the medical institution, the determination result data D5 including the information related to the determination result for the user determined to have the abnormal indication. The determination result data D5 is provided to the medical institution by the analysis device 50A transmitting the determination result data D5 to the medical institution terminal 70 provided in the medical institution. The mobility server 40A and the medical server 40B constitute a storage unit in the abnormality determination system 1 according to the present embodiment. In the storage unit, the mobility server 40A stores user data D2 related to the user of the vehicle V, and the medical server 40B stores vehicle data D1 including traveling information D13 of the vehicle V The mobility server 40A and the medical server 40B cooperate with each other to associate and manage the user data D2 and the vehicle data D1. The determination device 50B in the determination and analysis device constitutes a determination unit that determines the presence or absence, the content, and the like of the abnormal indication of the user from the vehicle data D1, and stores the determination result data D5 including the information related to the determination result in the storage unit (medical server 40B). The analysis device 50A constitutes a notification unit that transmits at least the determination result data D5 to the medical institution terminal 70 of the medical institution, receives the medical information data D6 created based on the determination result data D5 and the opinion of the medical worker in the medical institution from the medical institution terminal 70, and notifies the user terminal 60 of the medical information including the content based on the medical information data D6. In this case, the notification unit (analysis device 50A) may be configured to transmit the determination result data D5 and the user data D2 in association with each other to the medical institution terminal 70 provided in the medical institution if the determination unit (determination device 50B) determines that there is an abnormal indication in the user. In this way, the determination and analysis device includes the determination unit and the notification unit.

In the present embodiment, the medical institution terminal 70 is typically a terminal device installed at a medical institution such as a university hospital or a general hospital. The medical institution terminal 70 receives the determination result data D5 transmitted from the analysis device 50A. The medical institution terminal 70 can process determination result information D51, which is information related to the determination result included in the determination result data D5, to be utilizable for the medical worker such as a doctor of the medical institution, and display the determination result information D51 on a display unit or the like. The determination result information D51 includes, for example, information related to a degree of the abnormal indication of the user and a specific content of the abnormal indication. The determination result information D51 may include information related to biological information when the abnormal indication appears in the user.

For example, based on the determination result data D5 of the medical institution terminal 70, the medical worker such as a doctor of the medical institution confirms the degree of the abnormal indication of the user, the information related to the content of the abnormal indication of the user, the information related to the biological information of the user when it is determined that there is an abnormal indication in the user, and the like. Then, the doctor of the medical institution determines the presence or absence of the indication or the symptom of the disease related to the cognitive function of the user based on the content of the confirmed information and his or her own opinion. The doctor of the medical institution creates the medical information data D6 based on the content of the determination made by himself or herself, and stores the medical information data D6 in the medical institution terminal 70.

The medical information data D6 stored in a storage unit of the medical institution terminal 70 is transmitted to the service providing device 30 by the medical institution terminal 70. The transmission of the medical information data D6 from the medical institution terminal 70 to the service providing device 30 may be automatically performed every time the medical information data D6 is stored in the medical institution terminal 70, or may be automatically performed at a constant cycle (for example, every several hours). The transmission of the medical information data D6 from the medical institution terminal 70 to the service providing device 30 may be manually performed by the doctor in the medical institution or a person in charge other than the doctor.

The medical information data D6 includes, as the determination result related to the cognitive function of the user, the information related to the presence or absence of the indication or the symptom of the disease related to the cognitive function of the user determined by the medical worker such as a doctor of the medical institution. The medical information data D6 includes, for example, as a treatment program, information related to therapy determined to be suitable for the user by the medical worker such as a doctor. For example, a doctor as the medical worker selects therapy considered to be suitable for the user from medication therapy and occupational therapy such as exercise therapy, reminiscence therapy, music therapy, and cognitive stimulation therapy, and stores, in the storage unit of the medical institution terminal 70, information related to the therapy included in the medical information data D6 as a treatment program. The medical information data D6 includes information related to a consultation recommendation to the medical institution or a consultation recommendation on a cognitive function inspection as a consultation recommendation on the disease related to the cognitive function. The consultation recommendation for the disease related to the cognitive function is information related to the consultation recommendation for a specific medical institution determined to be suitable for the user by the medical worker such as a doctor. The selection of the medical institution in the consultation recommendation is not limited to an introduction by the doctor, and the analysis device 50A may select the medical institution from the user data D2 or the like on the basis of a residence, a medical history, or the like of the user, add the selected medical institution to a medical information notification, and transmit the medical information notification to the user terminal 60.

In response to receiving the medical information data D6 from the medical institution terminal 70, the analysis device 50A issues the medical information notification including the content based on the medical information data D6 to the user. In the present embodiment, the user terminal 60 typically includes a first user terminal 60A owned by the user himself or herself and a second user terminal 60B owned by a family of the user, a related person, or the like.

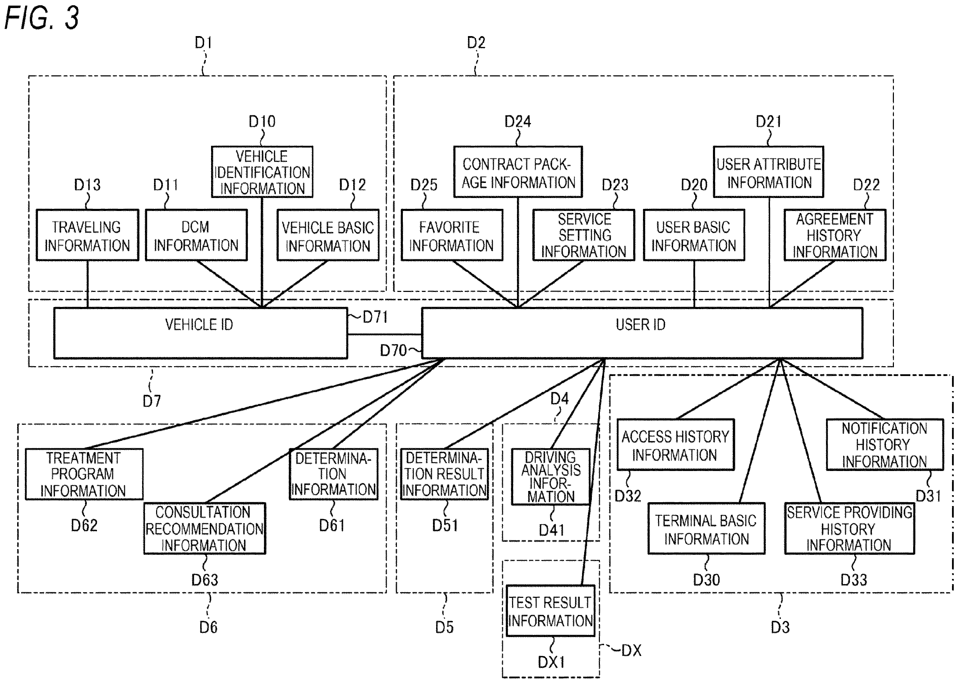

Hereinafter, configurations of the abnormality determination system 1 will be described in detail with reference to the drawings. In the following, first, an overview of main data treated by the abnormality determination system 1 will be described with reference to FIG. 3, and thereafter, the configurations of the abnormality determination system 1 will be described in detail with reference to other drawings and the like.

In the abnormality determination system 1, the vehicle V to be treated may be any driving system such as a gasoline vehicle, a diesel vehicle, a hybrid electric vehicle (HEV), a plug-in hybrid electric vehicle (PHEV), or an electric vehicle (EV). A driving method of the vehicle V may be any of manual driving, semi-automatic driving, fully automatic driving, and the like. In addition to an own vehicle of the user who can enjoy a service provided by the abnormality determination system 1, the vehicle V may be any one of a truck, a bus, a taxi, a special vehicle, and the like that can be specified as being used by the user

### <Main Data Treated by Abnormality Determination System>

The main data treated by the abnormality determination system 1 includes, for example, the vehicle data D1, the user data D2, terminal data D3, driving analysis data D4, the determination result data D5, the medical information data D6, test result data DX, and ID data D7 as shown in FIG. 3. The vehicle data D1, the user data D2, the terminal data D3, the driving analysis data D4, the determination result data D5, the medical information data D6, and the test result data DX are associated with the ID data D7 and the like and data-managed in the abnormality determination system 1.

### <Vehicle Data>

The vehicle data D1 is data related to the vehicle V Typically, the vehicle data D1 is data collected by the in-vehicle device 10 mounted on the vehicle V and transmitted to a service providing device 30 side. Here, the vehicle data D1 includes, for example, data related to vehicle identification information D10, DCM information D11, vehicle basic information D12, and the traveling information D13.

The vehicle identification information D10 is unique information assigned to each vehicle V to identify the vehicle V, and is, for example, information such as a vehicle identification number (VIN). In addition, the vehicle identification information D10 may be information such as a vehicle registration number (so-called car number) or a frame number of a vehicle. The DCM information D11 is information such as a type or an international mobile equipment identifier (IMEI) of a data communication module (DCM) mounted on the vehicle V The vehicle basic information D12 is information such as a vehicle name, a vehicle type, a model, and a grade of the vehicle V The traveling information D13 is information related to a traveling situation of the vehicle V In the present embodiment, the traveling information D13 includes driving information, vehicle state information, vehicle position information, device operation information, and image information.

The driving information is basic information related to driving of the vehicle V, and is, for example, information such as a vehicle speed, acceleration/deceleration, fuel consumption, an accumulated traveling distance, and a battery remaining amount. The driving information may include information related to the weather during traveling or information related to sound in the vehicle during traveling. The vehicle state information is detailed information related to a state of the vehicle V, and is, for example, information such as a steering wheel steering angle, an engine coolant temperature, an accelerator opening degree (accelerator operation amount), a brake opening degree (accelerator operation amount), an engine rotation speed, an engine torque, a motor torque, and a motor rotation speed. The vehicle position information is information such as position information (GNSS information, GPS information) of the vehicle V The device operation information is information related to an operation state of a device mounted on the vehicle V, and is, for example, information such as a door lock opening and closing state, a hazard lamp lighting state, a direction indicator operation state, a vehicle warning lamp lighting state, and presence or absence of an emergency notification. The image information is, for example, information such as a moving image inside and outside the vehicle captured in the vehicle V In addition, for example, information such as an acquisition date and time of information is added to the vehicle data D1. In addition to the above, the vehicle data D1 may include, for example, data related to the biological information of the user (driver) of the vehicle V The biological information of the user may include, in addition to various kinds of physiological information issued by a living body (user), various kinds of information derived from the physiological information, and is, for example, vital sign information such as a heart rate, a respiratory rate, a pulse rate, a blood pressure, and a body temperature, biological identification information such as a fingerprint, a voiceprint, and an iris, and information such as a blood alcohol concentration and an arousal level.

### <User Data>

The user data D2 is data related to the user of the vehicle V Here, the user in the abnormality determination system 1 is the driver of the vehicle V, and when the abnormality determination system 1 determines the presence or absence, the content, and the like of the abnormal indication and determines that there is an abnormal indication, the user receives the medical information notification including the content created based on the determination result and the opinion of the medical worker of the medical institution. A service utilizer who enjoys a service provided by the abnormality determination system 1 or a contractor who makes a contract for receiving the service is also included in the user who uses the abnormality determination system 1. That is, the user data D2 can also be referred to as contractor data related to the service or customer data for a business operator who develops the service. Typically, the user data D2 is data registered in the mobility server 40A by the in-vehicle device 10, the user terminal 60, or the like, or data automatically generated on a mobility server 40A side based on the data registered in the mobility server 40A. Here, the user data D2 includes, for example, data related to user basic information D20, user attribute information D21, agreement history information D22, service setting information D23, contract package information D24, favorite information D25, and the like.

The user basic information D20 is information related to the contractor (customer) registered by the user himself or herself, and is, for example, information such as a user name, a postal code, an address, a mail address, a telephone number, a work contact address, a personal number (my number) of the user, and an insurer number Here, the personal number (my number) is an identification number of an individual and is a number given to a person having a resident card in Japan based on the law in Japan. The insurer number is a number assigned to an insurer of a medical insurance for which the user insures, and is a number capable of identifying the insurer.

The user attribute information D21 is information related to an attribute of the user registered by the user himself or herself or automatically generated on the mobility server 40A side, and is, for example, information such as age, gender, occupation, and nationality. The agreement history information D22 is information related to various agreement histories automatically generated on the mobility server 40A side, and is, for example, information related to presence or absence of agreement with respect to a privacy policy or a service policy. Here, the agreement history information D22 includes information related to the presence or absence of agreement of the user in providing the vehicle data D1, the user data D2, the determination result data D5, and the like to the medical institution.

The service setting information D23 is setting information necessary for enjoying a service registered by the user himself or herself, and is, for example, information such as a language setting and a notification setting in various services. Here, the service setting information D23 includes terminal registration information of the user terminal 60. The terminal registration information of the user terminal 60 is information related to the user terminal 60 registered by the user himself or herself or automatically generated on the mobility server 40A side, and is information capable of specifying the user terminal 60 of a notification destination when the determination device 50B issues the medical information notification to the user. The contract package information D24 is information related to a contract automatically generated on the mobility server 40A side, and is, for example, information such as a contract status (valid/expiration/cancellation), a contract start date/expiration date/cancellation date, and a contract plan. The favorite information D25 is information related to a preference of the user registered by the user himself or herself, and is, for example, information related to a nearest comprehensive hospital, a medical institution that the user wants to have a consultation, a home hospital, an insurance company that is under contract, and a favorite agency (dealer). In addition, for example, information such as an acquisition date and time of the information is added to the user data D2.

### <Terminal Data>

The terminal data D3 is data related to a transmission history of the medical information notification to the user terminal 60, a transmission history of the determination result data D5 to the medical institution terminal 70, and a history of receiving the medical information data D6 from the medical institution terminal 70. Typically, the terminal data D3 is data collected in the service providing device 30 in accordance with transmission of data to the user terminal 60 and the medical institution terminal 70 and reception of the medical information data D6 from the medical institution terminal 70. The terminal data D3 includes, for example, data related to terminal basic information D30, notification history information D31, access history information D32, service providing history information D33, and the like.

The terminal basic information D30 is basic information capable of specifying the user terminal 60 and the medical institution terminal 70, and includes information such as a type of each terminal, an owner of each terminal, and an institution that owns each terminal. The notification history information D31 is information related to a transmission history when the determination result data D5 is transmitted from the service providing device 30 to the medical institution terminal 70. The access history information D32 is information related to an access history from the medical institution terminal 70 to the service providing device 30 and a reception history when the medical information data D6 in the service providing device 30 is received. The service providing history information D33 is information related to a providing history of the service provided by the service providing device 30 to the user (user terminal 60). The service providing history information D33 in the present embodiment is information related to a history in which the service providing device 30 notifies the user of the medical information having the content based on the medical information data D6. In addition, for example, information such as an acquisition date and time of the information is added to the terminal data D3.

### <Driving Analysis Data>

The driving analysis data D4 is data related to the driving situation of the user which is generated by the determination device 50B analyzing the vehicle data D1. The driving analysis data D4 includes driving analysis information D41. The driving analysis information D41 is information related to an analysis result of the vehicle data D1 obtained by the determination device 50B. For example, the driving analysis information D41 includes information related to the presence or absence, the content, and the like of the abnormal driving of the user in the vehicle data D1. The driving analysis information D41 includes, for example, history information related to a driving behavior such as sudden braking, sudden steering, and overlooking of a temporary stop sign. For example, the driving analysis information D41 includes information related to the number of times of sudden braking, sudden steering, overlooking of a temporary stop sign, and the like within a predetermined period (for example, within a period of traveling 100 km). The driving analysis information D41 may include the inter-vehicle distance when the inter-vehicle distance to the preceding vehicle is shortened and a surrounding situation of the vehicle V when the inter-vehicle distance to the preceding vehicle is shortened. Furthermore, the driving analysis information D41 may include the biological information of the user (driver). Here, the biological information included in the driving analysis information D41 may include, in addition to various kinds of physiological information issued by the living body (user), various kinds of information derived from the physiological information, and is, for example, the vital sign information such as a heart rate, a respiratory rate, a pulse rate, a blood pressure, and a body temperature, the biological identification information such as a fingerprint, a voiceprint, and an iris, and the information such as a blood alcohol concentration and an arousal level. The driving analysis information D41 may include information such as a date and time when the abnormal driving of the user is detected.

### <Determination Result Data>

The determination result data D5 is data generated by the determination device 50B performing determination related to the abnormal indication of the user based on the vehicle data D1. The determination device 50B applies the reference created based on the correlation relation between the driving operation performed on the vehicle V and the cognitive function state of the user to the driving analysis data D4, determines the presence or absence, the content, and the like of the indication (abnormal indication) of deterioration in cognitive function of the user who drives the vehicle V from the driving analysis data D4, and generates the determination result as the determination result data D5. The determination result data D5 includes the determination result information D51. The determination result information D51 includes, for example, information related to detailed contents of the abnormal indication of the user, such as a degree of the abnormal indication of the user, the driving behavior in which the abnormal indication of the user is frequently confirmed, and a time zone in which the abnormal indication of the user is frequently confirmed. The determination result information D51 may include the biological information of the user when the abnormal indication appears in the user. The determination result information D51 may include the information such as the date and time when the abnormal indication appears in the user.

### <Medical Information Data>

The medical information data D6 is data created based on the determination result data D5 and the opinion of the medical worker such as a doctor in the medical institution. The medical information data D6 includes, for example, determination information D61, treatment program information D62, and consultation recommendation information D63. The determination information D61 is information related to a determination result related to the cognitive function of the user and obtained by the medical worker such as a doctor of the medical institution. The determination information D61 includes, for example, information related to the presence or absence, the content, and the like of the indication or the symptom of the disease related to the cognitive function of the user. The treatment program information D62 is information related to a therapy determined to be suitable for the user by the medical worker such as a doctor of the medical institution. The treatment program information D62 includes, for example, information related to therapy selected by the medical worker from the medication therapy and the occupational therapy such as exercise therapy, reminiscence therapy, music therapy, and cognitive stimulation therapy. Furthermore, the treatment program information D62 may include, for example, information such as a specific content of the therapy selected by the medical worker, a location of a medical institution where the user can consult the therapy, an assumed treatment schedule, and a type of medicine considered to be suitable for the user. The consultation recommendation information D63 is information related to the consultation recommendation for the disease related to the cognitive function. The consultation recommendation information D63 includes, for example, information related to the consultation recommendation to the medical institution or the consultation recommendation of the cognitive function inspection. The consultation recommendation information D63 is, for example, information related to the consultation recommendation to the specific medical institution determined to be suitable for the user by the medical worker.

### <Test Result Data>

The test result data DX is data created based on a result of taking a cognitive function test performed on the user and provided from the determination device 50B. The determination device 50B stores cognitive function determination data D0 as information utilized for performing the cognitive function test, and the user can take the cognitive function test by accessing and executing the cognitive function determination data D0 stored in the determination device 50B from the user terminal 60. The cognitive function test simply tests whether the cognitive function is deteriorated, for example, a mini-mental state examination inspection (MMSE inspection) or a response to a check list indicating whether the symptom corresponds to a specific symptom of dementia. The test result data DX includes, for example, test result information DX1. The test result information DX1 is information related to the result of the cognitive function test of the user, and is, for example, information representing the degree of deterioration in cognitive function of the user by a numerical value or the like.

### <ID Data>

The ID data D7 is an identifier that can manage the vehicle data D1, the user data D2, the terminal data D3, the driving analysis data D4, the determination result data D5, the medical information data D6, and the test result data DX described above in association with each other. The ID data D7 includes, for example, data related to a user ID D70 and a vehicle ID D71. The user ID D70 is a unique identifier assigned to each user to identify the user. The user data D2, the terminal data D3, the driving analysis data D4, the determination result data D5, the medical information data D6, and the test result data DX described above are managed in association with the user ID D70. On the other hand, the vehicle ID D71 is a unique identifier assigned to each vehicle V in order to identify the vehicle V Here, in order to facilitate identification and management of the vehicle V, the vehicle ID D71 is set separately from the vehicle identification information D10 described above. The vehicle data D1 described above is managed in association with the vehicle ID D71.

The user ID D70 and the vehicle ID D71 are associated with each other in correspondence with the vehicle V used by the user. For example, when one user owns a plurality of vehicles V, a relation between the user ID D70 and the vehicle ID D71 is that a plurality of vehicle IDs D71 are associated with one user ID D70. As a result, the vehicle data D1, the user data D2, the terminal data D3, the driving analysis data D4, the determination result data D5, the medical information data D6, and the test result data DX can be associated with each other for each vehicle corresponding to each vehicle ID D71 based on the user ID D70 and the like. That is, the vehicle data D1 related to the vehicle V used by a specific user, the user data D2 related to the specific user, the terminal data D3, the driving analysis data D4, the determination result data D5, the medical information data D6, and the test result data DX can be associated with each other via the user ID D70 and the vehicle ID D71 and can be managed as a data set. When the plurality of vehicle IDs D71 are associated with one user ID D70 as described above, a plurality of pieces of vehicle data D1 are respectively associated with the user data D2 of the corresponding user, the terminal data D3, the driving analysis data D4, the determination result data D5, the medical information data D6, and the test result data DX via the user ID D70 and the vehicle IDs D71. On the other hand, when one vehicle V is used by a plurality of users, a relation between the user ID D70 and the vehicle ID D71 is that a plurality of user IDs D70 are associated with one vehicle ID D71.

An overview of the main data treated by the abnormality determination system 1 has been described above. Although the abnormality determination system 1 also treats data other than the data described above, the data will be appropriately described later.

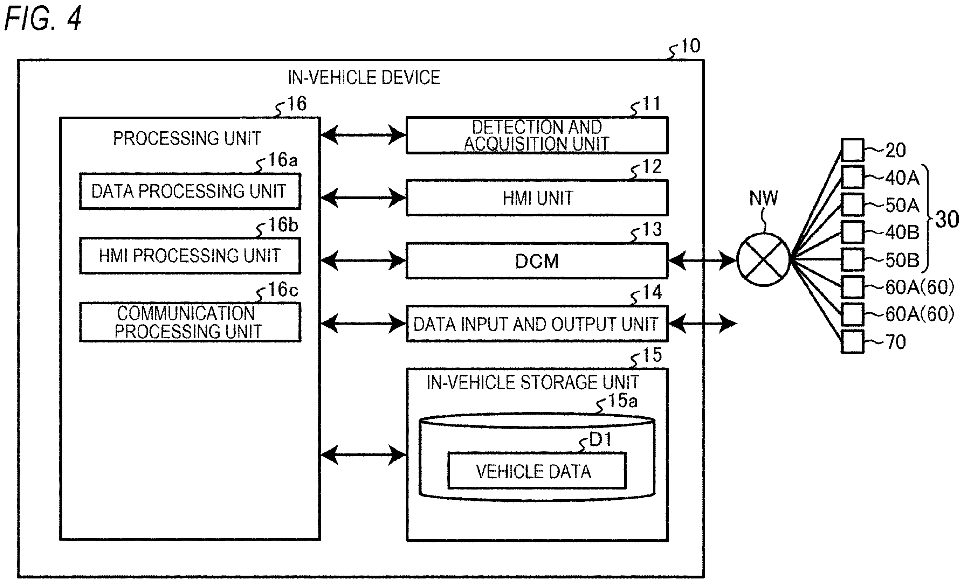

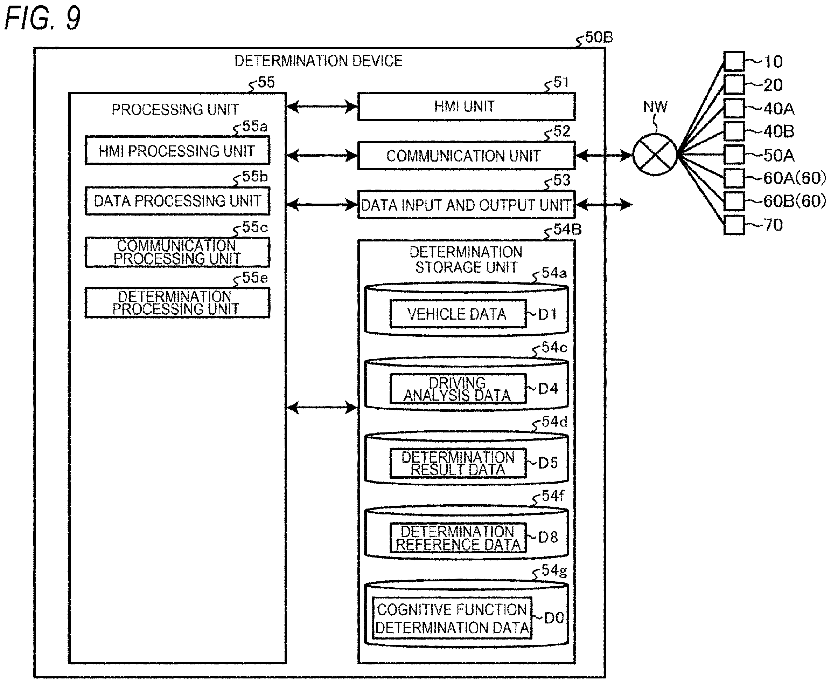

Next, configurations of the abnormality determination system 1 will be described with reference to FIGS. 4 to 15. In the following description, various kinds of data may be appropriately referred to in FIG. 3.

### <Basic Configuration of In-vehicle Device>

The in-vehicle device 10 shown in FIG. 4 is a device that is mounted on the vehicle V and that collects and stores the vehicle data D1 related to the vehicle V in association with the vehicle identification information D10 of the vehicle V The in-vehicle device 10 is capable of communicating with an external device of the vehicle V For example, the in-vehicle device 10 may include a function of a so-called drive recorder or the like that is mounted on the vehicle V and records various kinds of information, and may be configured to be installed in the vehicle V later.

Specifically, as shown in FIG. 4, the in-vehicle device 10 includes a detection and acquisition unit 11, a human machine interface (HMI) unit 12, a DCM 13, a data input and output unit 14, an in-vehicle storage unit 15, and a processing unit 16. The detection and acquisition unit 11, the HMI unit 12, the DCM 13, the data input and output unit 14, the in-vehicle storage unit 15, and the processing unit 16 are communicably connected to each other.

The detection and acquisition unit 11 is a device that detects and acquires various kinds of information included in the vehicle data D1 related to the vehicle V The detection and acquisition unit 11 detects and acquires the vehicle identification information D10, the DCM information D11, the vehicle basic information D12, the traveling information D13, and the like as the vehicle data D1. The detection and acquisition unit 11 includes, for example, various sensors provided in units of the vehicle V, a detector, a GPS receiver, a microphone, and an in-vehicle camera. The detection and acquisition unit 11 may include, for example, a device that acquires various kinds of information from an electronic control unit (ECU) that integrally controls the entire vehicle V When the biological information or the like of the user (driver) of the vehicle V is included in the vehicle data D1, the detection and acquisition unit 11 may include a device that acquires various kinds of information in cooperation with a monitoring device that monitors the user or a wearable terminal worn by the user.

The HMI unit 12 is a device that performs various inputs and outputs in the in-vehicle device 10. The HMI unit 12 may include, for example, a keyboard, a switch, an input button, a mouse pointer, a trackball, a joystick, a touch pad, a microphone, and a voice input device as an operation input device that performs various inputs to the in-vehicle device 10. The HMI unit 12 is implemented as an information output device that performs various outputs from the in-vehicle device 10. Here, the information output device is a touch panel device or the like capable of outputting information to a user and receiving an operation input from the user. The HMI unit 12 may include, for example, a speaker and a display as an information output device that performs various outputs from the in-vehicle device 10.

The DCM 13 is a communication module capable of communicating with an external device of the vehicle V The DCM 13 is communicably connected to the network NW by wireless communication, and communicates with the external device of the vehicle V (typically, data processing device 20) via the network NW.

The data input and output unit 14 is a data input and output device that inputs and outputs data (information) to and from the in-vehicle device 10. The data input and output unit 14 is implemented by a recording medium interface or the like, and reads and writes various kinds of data from and to a recording medium such as a flexible disk (FD), a solid state drive (SSD), a hard disk drive (HDD), a magneto-optical disk, a USB memory, an SD card memory, a flash memory, a CD-ROM, or a DVD.

The in-vehicle storage unit 15 is a storage circuit that stores various kinds of data. The in-vehicle storage unit 15 may be, for example, a storage device having a relatively large capacity such as an HDD, an SSD, or an optical disk, or a semiconductor memory capable of rewriting data such as a RAM, a flash memory, or a non volatile static random access memory (NVSRAM). The in-vehicle storage unit 15 stores, for example, programs that enable units of the in-vehicle device 10 to implement various functions. The in-vehicle storage unit 15 stores various kinds of data necessary for various kinds of processes in the processing unit 16, and the processing unit 16 or the like reads the various kinds of data as necessary.

The in-vehicle storage unit 15 according to the present embodiment includes a vehicle data storage unit 15a in terms of functional concept.

The vehicle data storage unit 15a is a storage area for storing the vehicle data D1 in the in-vehicle storage unit 15. The vehicle data storage unit 15a stores, as the vehicle data D1, data related to the vehicle identification information D10, the DCM information D11, the vehicle basic information D12, the traveling information D13, and the like detected and acquired by the detection and acquisition unit 11.

The processing unit 16 is a processing circuit that integrally controls the in-vehicle device 10 and implements various processing functions of the in-vehicle device 10. The processing unit 16 is implemented by, for example, a processor, a RAM, and a ROM. The processor refers to a circuit such as a central processing unit (CPU), a micro processing unit (MPU), a field programmable gate array (FPGA), or an application specific integrated circuit (ASIC). The processing unit 16 implements each processing function, for example, by executing a program read from the in-vehicle storage unit 15. For example, the processing unit 16 is capable of executing a process of detecting and acquiring the vehicle data D1 by the detection and acquisition unit 11 and storing the vehicle data D1 in the vehicle data storage unit 15a, a process of operating the HMI unit 12, a process of performing data communication via the DCM 13, and the like.

The processing unit 16 according to the present embodiment includes a data processing unit 16a, an HMI processing unit 16b, and a communication processing unit 16c in terms of functional concept in order to implement the various processing functions described above. The processing unit 16 implements processing functions of the data processing unit 16a, the HMI processing unit 16b, and the communication processing unit 16c, for example, by reading and executing the program (not shown) stored in the in-vehicle storage unit 15.

The data processing unit 16a is a unit having a function capable of executing a process related to various kinds of data in the in-vehicle device 10. The data processing unit 16a is capable of executing a process of detecting and acquiring information constituting the vehicle data D1 by the detection and acquisition unit 11. The data processing unit 16a is capable of executing a process of storing, in the vehicle data storage unit 15a, information detected or acquired by the detection and acquisition unit 11 as the vehicle data D1. The data processing unit 16a is capable of executing a process of reading the vehicle data D1 from the vehicle data storage unit 15a in response to a request. The data processing unit 16a is capable of executing a process of inputting or outputting data to or from the in-vehicle device 10 via the data input and output unit 14. The data processing unit 16a is also capable of executing a process of storing data input via the data input and output unit 14 in the in-vehicle storage unit 15 and a process of reading, from the in-vehicle storage unit 15, data output via the data input and output unit 14. The data processing unit 16a is also capable of executing a process of storing data registered in accordance with an operation on an operation input device constituting the HMI unit 12 in the in-vehicle storage unit 15 and a process of reading the data from the in-vehicle storage unit 15.

For example, the data processing unit 16a may control the detection and acquisition unit 11 to detect and acquire information constituting the vehicle data D1 at a predetermined sampling cycle, or may detect and acquire the information at an appropriate timing. The data processing unit 16a may acquire a part of the vehicle data D1, such as the vehicle identification information D10, the DCM information D11, and the vehicle basic information D12, registered in accordance with an operation on the operation input device constituting the HMI unit 12. When storing the vehicle data D1 in the vehicle data storage unit 15a, the data processing unit 16a stores the vehicle identification information D 10 of the vehicle V on which the in-vehicle device 10 is mounted and the vehicle data D 1 in association with each other in the vehicle data storage unit 15a. In this case, for example, the data processing unit 16a can store, in the vehicle data storage unit 15a, the vehicle data D1 along a time series together with information on a date and time when the vehicle data D1 is collected or the like.

The HMI processing unit 16b is a unit having a function capable of executing a process of controlling the HMI unit 12 and operating the HMI unit 12. The HMI processing unit 16b is capable of executing a process of controlling the operation input device constituting the HMI unit 12 and inputting an operation via the operation input device. The HMI processing unit 16b is capable of executing a process of controlling the information output device constituting the HMI unit 12 and outputting information via the information output device.

The communication processing unit 16c is a unit having a function capable of executing a process of controlling the DCM 13 and performing data communication. The communication processing unit 16c is capable of executing a process of transmitting and receiving data to and from other devices such as the data processing device 20 via the DCM 13. More specifically, the communication processing unit 16c is capable of executing a process of transmitting the vehicle data D1 to the data processing device 20 via the DCM 13 in a state where the vehicle identification information D10 and the corresponding vehicle data D1 are associated with each other. The communication processing unit 16c is capable of executing a process of receiving various commands, requests, and various kinds of information from the data processing device 20 via the DCM 13. The communication processing unit 16c is capable of executing a process of transmitting various commands, requests, and various kinds of information to the data processing device 20 via the DCM 13.

For example, the communication processing unit 16c can transmit the vehicle data D 1 stored in the vehicle data storage unit 15a to the data processing device 20 at an appropriate timing. In this case, for example, the communication processing unit 16c may sequentially transmit the vehicle data D1 to the data processing device 20 in real time in accordance with a sampling cycle of the detection and acquisition unit 11, or may collectively transmit the vehicle data D1 of a plurality of sampling cycles to the data processing device 20 at a cycle different from the sampling cycle of the detection and acquisition unit 11. For example, the communication processing unit 16c may transmit the vehicle data D1 to the data processing device 20 at any timing in accordance with an operation on the operation input device constituting the HMI unit 12, or may transmit the vehicle data D1 to the data processing device 20 at a timing when a request such as a transmission command from an external device (data processing device 20 or the like) is received. In this case, for example, the in-vehicle device 10 can be expected to have an effect of reducing congestion due to an increase in a communication traffic amount. For example, the communication processing unit 16c can change a transmission cycle of the vehicle data D1 according to the state of the vehicle V In this case, for example, when the vehicle V is stopped, a significant change in the vehicle speed, the position information, or the like is not expected, and thus the communication processing unit 16c may transmit the vehicle data D1 at a transmission cycle that is relatively longer than a transmission cycle when the vehicle V is traveling. In this case, for example, the in-vehicle device 10 can transmit data at an appropriate timing in accordance with the state of the vehicle V

### <Basic Configuration of Data Processing Device>

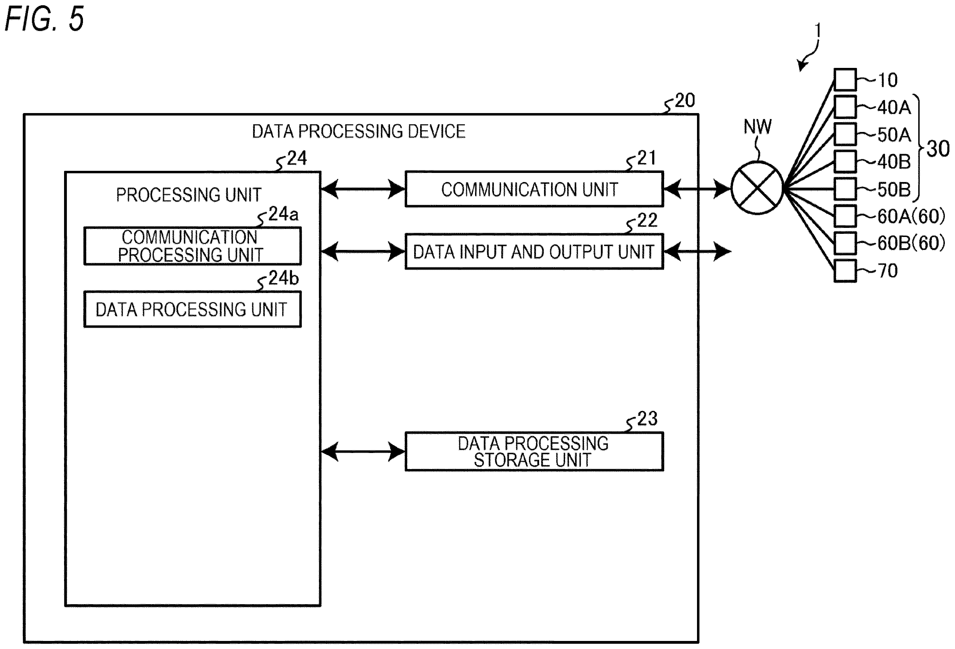

The data processing device 20 shown in FIG. 5 is a network relay device that relays data communication between the in-vehicle device 10 and the service providing device 30 (for example, mobility server 40A and medical server 40B). In the present embodiment, the data processing device 20 relays data to be transmitted from the in-vehicle device 10 to the service providing device 30. The service providing device 30 stores the data received from the data processing device 20 in the mobility server 40A and the medical server 40B. The data processing device 20 may be implemented by, for example, various computer devices. The data processing device 20 can also be implemented by, for example, installing programs for implementing various processes in a known computer device.

Specifically, as shown in FIG. 5, the data processing device 20 includes a communication unit 21, a data input and output unit 22, a data processing storage unit 23, and a processing unit 24. The communication unit 21, the data input and output unit 22, the data processing storage unit 23, and the processing unit 24 are communicably connected to each other.

The communication unit 21 is a communication module capable of communicating with devices other than the data processing device 20. The communication unit 21 is communicably connected to the network NW regardless of wireless communication or wired communication, and communicates with other devices (for example, in-vehicle device 10 and service providing device 30) via the network NW.

The data input and output unit 22 is a data input and output device that inputs and outputs data (information) to and from the data processing device 20. The data input and output unit 22 has substantially the same configuration as the data input and output unit 14 described above, and is implemented by a recording medium interface or the like.

The data processing storage unit 23 is a storage circuit that stores various kinds of data. The data processing storage unit 23 has substantially the same configuration as the in-vehicle storage unit 15 described above, and is implemented by, for example, an HDD, an SSD, an optical disk, and a semiconductor memory. The data processing storage unit 23 stores, for example, a program that enables each unit of the data processing device 20 to implement various functions. The data processing storage unit 23 stores various kinds of data necessary for various kinds of processes in the processing unit 24, and the processing unit 24 or the like reads the various kinds of data as necessary

The processing unit 24 is a processing circuit that integrally controls the data processing device 20 and implements various processing functions of the data processing device 20. The processing unit 24 has substantially the same configuration as the processing unit 16 described above, and is implemented by, for example, a processor, a RAM, and a ROM. The processing unit 24 implements processing functions, for example, by reading and executing the programs stored in the data processing storage unit 23. For example, the processing unit 24 is capable of executing a process of performing data communication via the communication unit 21, a process of relaying various kinds of data, a process of transmitting data received from the in-vehicle device 10 to the mobility server 40A and the medical server 40B and storing the data, and the like.

The processing unit 24 according to the present embodiment includes a communication processing unit 24a and a data processing unit 24b in terms of functional concept in order to implement the various processing functions described above. The processing unit 24 implements processing functions of the data processing unit 24b and the communication processing unit 24a by reading and executing a program stored in the data processing storage unit 23, for example.

The communication processing unit 24a is a unit having a function capable of executing a process of controlling the communication unit 21 and performing data communication. The communication processing unit 24a is capable of executing a process of transmitting and receiving data to and from other devices such as the in-vehicle device 10 and the service providing device 30 via the communication unit 21. More specifically, for example, the communication processing unit 24a is capable of executing a process of receiving the vehicle data D1 from the in-vehicle device 10 via the communication unit 21. The communication processing unit 24a is capable of executing a process of transmitting the vehicle data D1 to the service providing device 30 (for example, mobility server 40A and medical server 40B) via the communication unit 21. The communication processing unit 24a is capable of executing a process of receiving various commands and requests from the in-vehicle device 10, the service providing device 30, and the like via the communication unit 21. The communication processing unit 24a is capable of executing a process of transmitting various commands, requests, and various kinds of information to the in-vehicle device 10 and the service providing device 30 (for example, the mobility server 40A and the medical server 40B) via the communication unit 21.

For example, when relaying data between the devices, the communication processing unit 24a executes processes such as a process of blocking unauthorized communication, a process of ensuring communication with a high security level such as encrypted communication, a routing process of distributing a transmission destination of data, and a protocol conversion process of converting a communication protocol between different networks.

The data processing unit 24b is a unit having a function capable of executing a process related to various kinds of data in the data processing device 20. The data processing unit 24b is capable of executing a process of storing data received via the communication unit 21 in the data processing storage unit 23. The data processing unit 24b is capable of executing a process of reading data transmitted via the communication unit 21 from the data processing storage unit 23. The data processing unit 24b is capable of executing a process of inputting and outputting data to and from the data processing device 20 via the data input and output unit 22. The data processing unit 24b is also capable of executing a process of storing data input via the data input and output unit 22 in the data processing storage unit 23 and a process of reading data output via the data input and output unit 22 from the data processing storage unit 23.

The communication processing unit 24a transmits the vehicle data D1 read from the data processing storage unit 23 by the data processing unit 24b to the service providing device 30 (mobility server 40A and medical server 40B) via the communication unit 21.

### <Basic Configuration of Service Providing Device>

The service providing device 30 collectively manages various kinds of data treated by the abnormality determination system 1, analyzes the driving situation of the user based on the vehicle data D1, and determines the presence or absence, the content, and the like of an abnormal indication of the user based on the driving analysis data D4 including information related to the analysis result. The service providing device 30 stores, in the storage unit, the determination result data D5 including the information related to the determination result of the abnormal indication of the user, and transmits the determination result data D5 and the user data D2 in association with each other to the medical institution terminal 70 provided in the medical institution if it is determined that the abnormal indication is present in the user. As shown in FIG. 2, the service providing device 30 includes the mobility server 40A, the medical server 40B, and the determination and analysis device (analysis device 50A and determination device 50B). Typically, the service providing device 30 constitutes a base station device provided at a base station that relays communication between the vehicle V and external terminals such as the user terminal 60 and the medical institution terminal 70. In the present embodiment, the base station device (service providing device 30) includes a first base station device disposed at a mobility base station 101 and including the mobility server 40A and the analysis device 50A, and a second base station device disposed at a medical base station 102 and including the medical server 40B and the determination device 50B. The mobility base station 101 is, for example, a base station managed by a developer or an analysis business operator of the vehicle V The medical base station 102 is, for example, a base station managed by a business operator that develops various services related to medical care. The determination and analysis device (analysis device 50A and determination device 50B) does not need to be disposed at the mobility base station 101 and the medical base station 102, and may be disposed at a location different from these base stations. That is, at least the mobility server 40A and the medical server 40B may constitute the base station device. In this case, the mobility server 40A constitutes the first base station device, and the medical server 40B constitutes the second base station device. In this case, for example, the analysis device 50A may be configured to access the first base station device from a remote location different from the location of the mobility base station 101, and the determination device 50B may be configured to access the second base station device from a remote location different from the location of the medical base station 102. The determination and analysis device may be one in which functions of the analysis device 50A and the determination device 50B are integrated into one device, and may be configured to access servers disposed at the mobility base station 101 and the medical base station 102 from a remote location different from the locations of these base stations.

In the service providing device 30 according to the present embodiment, the determination device 50B is configured to communicate with the mobility server 40A, the medical server 40B, and the user terminal 60. The medical server 40B is configured to communicate with the determination device 50B. The determination device 50B analyzes the driving situation of the user based on the vehicle data D1 acquired from the mobility server 40A and the medical server 40B, and generates the driving analysis data D4 including information related to the analysis result. Then, the determination device 50B determines the presence or absence, the content, and the like of the abnormal indication of the user based on the driving analysis data D4, and generates the determination result data D5 including the determination result. In the service providing device 30 according to the present embodiment, the analysis device 50A is configured to communicate with the mobility server 40A, the medical server 40B, the determination device 50B, and the medical institution terminal 70. The mobility server 40A is configured to communicate with the analysis device 50A and the user terminal 60. In the service providing device 30, the determination result data D5 is transmitted from the determination device 50B to the medical institution terminal 70 via the analysis device 50A. In the service providing device 30, the analysis device 50A receives the medical information data D6 from the medical institution terminal 70. The analysis device 50A notifies the user terminal 60 of the medical information including the content based on the medical information data D6 received from the medical institution terminal 70. In this case, the medical information is transmitted from the analysis device 50A to the user terminal 60 via the mobility server 40A. In this way, the service providing device 30 according to the present embodiment is configured such that the medical institution terminal 70 cannot directly access the mobility server 40A and the medical server 40B.

The mobility server 40A, the analysis device 50A, the medical server 40B, and the determination device 50B may be implemented by, for example, various computer devices. The mobility server 40A, the analysis device 50A, the medical server 40B, and the determination device 50B can also be implemented by, for example, installing programs for implementing various processes in a known computer device. In the present embodiment, the mobility server 40A and the medical server 40B are configured separately and independently as physically different servers. The mobility server 40A and the medical server 40B constitute the storage unit of the abnormality determination system 1 in cooperation with each other. The mobility server 40A and the medical server 40B manage various kinds of data in cooperation with each other. The mobility server 40A and the medical server 40B may be implemented by, for example, various computer devices. The mobility server 40A and the medical server 40B can also be implemented by, for example, installing programs for implementing various processes in a known computer device. Here, the mobility server 40A and the medical server 40B may not be limited to being separately and independently configured as different physical servers, or may be separately and independently configured as so-called virtual servers on the same physical server or a plurality of physical servers according to various virtualization techniques. That is, the mobility server 40A and the medical server 40B are configured to hold data independently of each other As described above, the mobility server 40A and the medical server 40B may be physically and structurally independent of each other, or may be configured such that, for example, each component such as a processor is physically common and storage areas are independent of each other.

The mobility server 40A and medical server 40B included in the service providing device 30 are typically configured as so-called relational servers that store various kinds of data in a table format such as CSV or Excel, but are not limited thereto. For example, the mobility server 40A and the medical server 40B may be configured as so-called No SQL servers that store various kinds of data in a format such as XML or JSON. In this case, for example, the mobility server 40A and the medical server 40B can be configured to easily search for a large amount of data at the same time.

### <Basic Configuration of Mobility Server>

The mobility server 40A is configured to cooperate with the medical server 40B, and is a server that mainly manages data used for a service provided to the user of the vehicle V The mobility server 40A and the medical server 40B function as connected servers that cooperate with each other to deploy connected services. In the abnormality determination system 1 according to the present embodiment, the data stored in the medical server 40B is used to perform determination related to the abnormal indication of the user, and a medical information notification service for transmitting the medical information notification to the user terminal 60 based on the detection result is provided to the user. In the abnormality determination system 1 according to the present embodiment, not only the medical information notification service, but also connected services such as contract management, parking position search, vehicle remote control, emergency notification support, trouble support, security notification, and driving history evaluation can be provided to the user by using the data stored in the mobility server 40A.

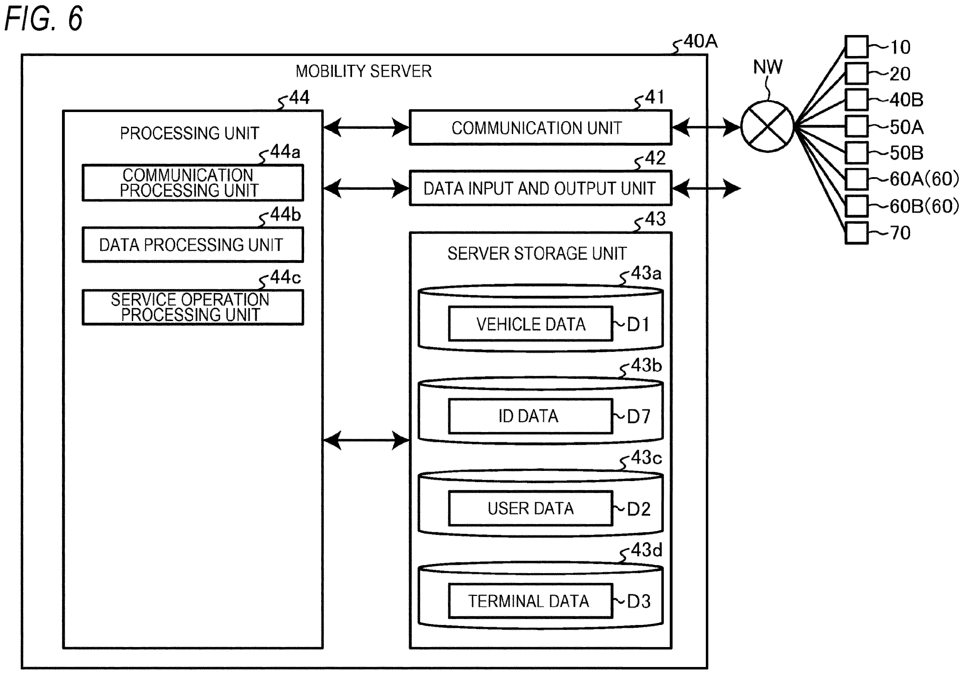

Specifically, as shown in FIG. 6, the mobility server 40A includes a communication unit 41, a data input and output unit 42, a server storage unit 43, and a processing unit 44. The communication unit 41, the data input and output unit 42, the server storage unit 43, and the processing unit 44 are communicably connected to each other.

The communication unit 41 is a communication module capable of communicating with devices other than the mobility server 40A. The communication unit 41 has substantially the same configuration as the communication unit 21 described above, is communicably connected to the network NW regardless of wireless communication or wired communication, and communicates with other devices (for example, data processing device 20, analysis device 50A, and user terminal 60) via the network NW.

The data input and output unit 42 is a data input and output device that inputs and outputs data (information) to and from the mobility server 40A. The data input and output unit 42 has substantially the same configuration as the data input and output unit 14 described above, and is implemented by a recording medium interface or the like.

The server storage unit 43 is a storage circuit that stores various kinds of data. The server storage unit 43 has substantially the same configuration as the in-vehicle storage unit 15 described above, and is implemented by, for example, an HDD, an SSD, an optical disk, and a semiconductor memory. The server storage unit 43 stores, for example, programs that enable units of the mobility server 40A to implement various functions (not shown). The server storage unit 43 stores various kinds of data necessary for various kinds of processes in the processing unit 44, and the processing unit 44 or the like reads the various kinds of data as necessary.

The server storage unit 43 according to the present embodiment includes a vehicle data storage unit 43a, an ID data storage unit 43b, a user data storage unit 43c, and a terminal data storage unit 43d in terms of functional concept.

The vehicle data storage unit 43a has substantially the same configuration as the in-vehicle storage unit 15 described above, and is implemented by, for example, an HDD, an SSD, an optical disk, and a semiconductor memory. The vehicle data storage unit 43a is a storage area for storing the vehicle data D1 in the server storage unit 43. The vehicle data storage unit 43a stores the vehicle data D1 transmitted from the in-vehicle device 10. The vehicle data storage unit 43a stores, for example, the vehicle data D1 including data related to the vehicle identification information D10, the DCM information D11, the vehicle basic information D12, the traveling information D13, and the like.

The ID data storage unit 43b is a storage area for storing the ID data D7 in the server storage unit 43. The ID data storage unit 43b stores, for example, the ID data D7 including data related to the user ID D70, the vehicle ID D71, and the like registered via the user terminal 60 or automatically generated by the processing unit 44 of the mobility server 40A.

The user data storage unit 43c is a storage area for storing the user data D2 in the server storage unit 43. The user data storage unit 43c stores, for example, the user data D2 including data related to the user basic information D20, the user attribute information D21, the agreement history information D22, the service setting information D23, the contract package information D24, the favorite information D25, and the like registered via the in-vehicle device 10 and the user terminal 60, or automatically generated by the processing unit 44 of the mobility server 40A.

The terminal data storage unit 43d is a storage area for storing the terminal data D3 in the server storage unit 43. The terminal data storage unit 43d stores, for example, the terminal data D3 including data related to the terminal basic information D30, the notification history information D31, the access history information D32, the service providing history information D33, and the like of the user terminal 60.

The processing unit 44 is a processing circuit that integrally controls the mobility server 40A and implements various processing functions of the mobility server 40A. The processing unit 44 has substantially the same configuration as the processing unit 16 described above, and is implemented by, for example, a processor, a RAM, and a ROM. The processing unit 44 implements processing functions, for example, by reading and executing the program stored in the server storage unit 43. For example, the processing unit 44 is capable of executing a process of performing data communication via the communication unit 41, a process of specifying the user data D2 corresponding to the determination result data D5 and the medical information data D6, a process of calculating various kinds of data, a process of providing various services to the user, and the like.

The processing unit 44 according to the present embodiment includes a communication processing unit 44a, a data processing unit 44b, and a service operation processing unit 44c in terms of functional concept in order to implement the various processing functions described above. The processing unit 44 implements processing functions of the communication processing unit 44a, the data processing unit 44b, and the service operation processing unit 44c, for example, by reading and executing the program stored in the server storage unit 43.

The communication processing unit 44a is a unit having a function capable of executing a process of controlling the communication unit 41 and performing data communication. The communication processing unit 44a is capable of executing a process of transmitting and receiving data to and from other devices such as the data processing device 20 and the user terminal 60 via the communication unit 41.

For example, the communication processing unit 44a is capable of executing a process of receiving the ID data D7 from the user terminal 60 or the like via the communication unit 41. The communication processing unit 44a is capable of executing a process of receiving the vehicle data D1 transmitted from the data processing device 20 to the mobility server 40A via the communication unit 41. The communication processing unit 44a is capable of executing a process of receiving the user data D2 from the in-vehicle device 10 and the user terminal 60 via the communication unit 41. The communication processing unit 44a is capable of executing a process of transmitting the medical information notification to the user terminal 60 via the communication unit 41 in response to a request from the analysis device 50A. The communication processing unit 44a is capable of executing a process of receiving various commands and requests from the data processing device 20, the analysis device 50A, the user terminal 60, and the like via the communication unit 41.

The data processing unit 44b is a unit having a function capable of executing a process related to various kinds of data in the mobility server 40A. The data processing unit 44b is capable of executing a process of storing data received via the communication unit 41 in the server storage unit 43. The data processing unit 44b is capable of executing a process of reading data transmitted via the communication unit 41 from the server storage unit 43. The data processing unit 44b is also capable of executing a process of storing data input via the data input and output unit 42 in the server storage unit 43 and a process of reading data output via the data input and output unit 42 from the server storage unit 43.

More specifically, for example, the data processing unit 44b is capable of executing a process of storing the vehicle data D1 transmitted from the in-vehicle device 10 via the data processing device 20 in the vehicle data storage unit 43a. The data processing unit 44b is capable of executing a process of reading the vehicle data D1 from the vehicle data storage unit 43a in response to a request.

The data processing unit 44b is capable of executing a process of storing, in the ID data storage unit 43b, data related to the user ID D70, the vehicle ID D71, or the like registered via the user terminal 60 or the like as the ID data D7. The data processing unit 44b may execute a process of automatically generating data related to the user ID D70, the vehicle ID D71, or the like based on information registered via the user terminal 60 or the like, and storing the generated data as the ID data D7 in the ID data storage unit 43b. The data processing unit 44b is capable of executing a process of reading the ID data D7 from the ID data storage unit 43b in response to the request.

The data processing unit 44b is capable of executing a process of storing, in the user data storage unit 43c, data related to the user basic information D20, the service setting information D23, the favorite information D25, and the like registered via the user terminal 60 or the like as the user data D2. The data processing unit 44b is capable of executing a process of automatically generating data related to a part of the user data D2 stored in the mobility server 40A, that is, the user attribute information D21, the agreement history information D22, the contract package information D24, and the like based on information registered via the user terminal 60 or the like, and storing the generated data as the user data D2 in the user data storage unit 43c. For example, the data processing unit 44b may store data registered via the in-vehicle device 10 as a part of the user data D2 in the user data storage unit 43c and form the user data D2. The data processing unit 44b is capable of executing a process of reading the user data D2 from the user data storage unit 43c in response to a request.

The data processing unit 44b is capable of executing a process of storing, in the terminal data storage unit 43d, data related to the terminal basic information D30 transmitted from the user terminal 60, the medical institution terminal 70, or the like as the terminal data D3. The data processing unit 44b is capable of executing a process of acquiring data related to the notification history information D31, the access history information D32, the service providing history information D33, and the like from the analysis device 50A or the like, generating the data as the terminal data D3, and storing the data in the terminal data storage unit 43d. The data processing unit 44b is capable of executing a process of reading the terminal data D3 from the terminal data storage unit 43d in response to a request.

As described above, when respectively storing the vehicle data D1, the user data D2, and the terminal data D3 in the vehicle data storage unit 43a, the user data storage unit 43c, and the terminal data storage unit 43d, the data processing unit 44b is capable of executing a process of associating the vehicle data D1, the user data D2, and the terminal data D3 with each other. For example, the data processing unit 44b associates, via the vehicle identification information D10 included in the vehicle data D1 and the user ID D70 and the vehicle ID D71 constituting the ID data D7, the vehicle data D1, the user data D2, and the terminal data D3 stored in the vehicle data storage unit 43a, the user data storage unit 43c, and the terminal data storage unit 43d with each other to form a data set.

That is, the server storage unit 43 described above stores the vehicle data D1 stored in the vehicle data storage unit 43a, the user data D2 stored in the user data storage unit 43c, and the terminal data D3 stored in the terminal data storage unit 43d by the data processing unit 44b associating the above data with each other based on the vehicle identification information D10 and the ID data D7, and stores the above data in a database as a data set.

The service operation processing unit 44c is a unit having a function capable of executing a process related to provision of a service based on commands and requests received from the data processing device 20, the analysis device 50A, the determination device 50B, and the like, and based on data stored in the server storage unit 43. For example, in the mobility server 40A, the service operation processing unit 44c specifies a user (user terminal) as a target of the medical information notification from the user data D2 corresponding to the medical information data D6 of the determination device 50B in response to a command or a request received from the analysis device 50A, and transmits the medical information notification to the user. For example, the service operation processing unit 44c is capable of executing some or all of the services such as the contract management, the parking position search, the vehicle remote control, the emergency notification support, the trouble support, the security notification, and the driving history evaluation described above. The service operation processing unit 44c may execute the services in cooperation with the user terminal 60 or other external terminals. When performing an operation related to provision of a service, the service operation processing unit 44c appropriately reads data necessary for executing the requested service from the vehicle data D1, the user data D2, and the terminal data D3 stored in the server storage unit 43, and operates to provide the service to the user based on the data.

### <Basic Configuration of Analysis Device>

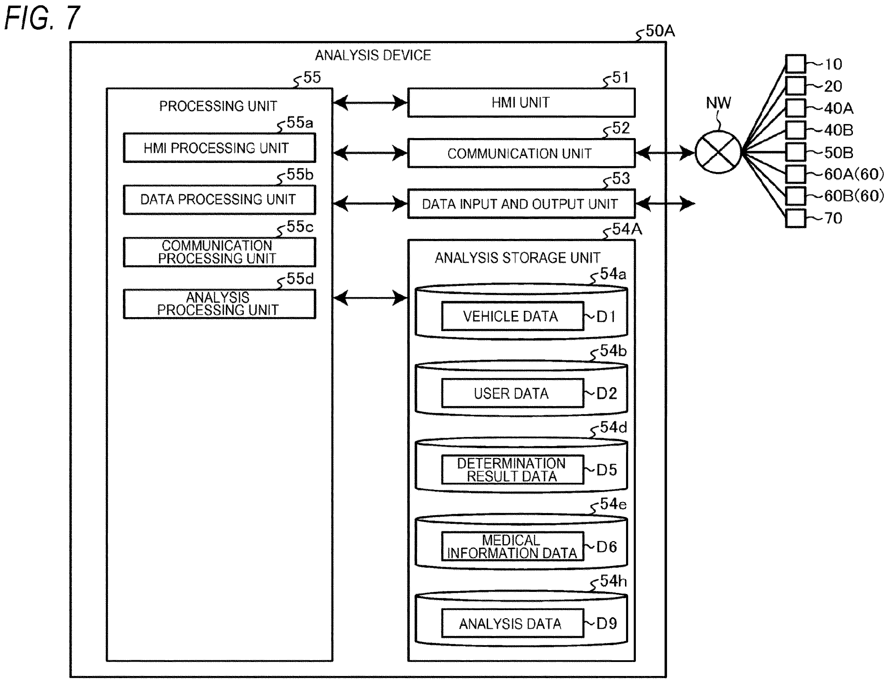

The analysis device 50A shown in FIG. 7 is configured to relay data transmission and reception between the mobility server 40A and the medical server 40B. The analysis device 50A is configured to communicate with the medical institution terminal 70 and the determination device 50B, and transmits the determination result data D5 received from the determination device 50B to the medical institution terminal 70. That is, the analysis device 50A functions as a relay in data transmission and reception between the determination device 50B and the medical institution terminal 70. The analysis device 50A receives, from the medical institution terminal 70, the medical information data D6 created based on the determination result data D5 and the opinion of the medical worker of the medical institution. The analysis device 50A notifies the user (user terminal 60) of the medical information including the content based on the medical information data D6 via the mobility server 40A. The analysis device 50A is capable of not only notifying the medical information but also performing various analyses (quality investigation, accident analysis, failure analysis, feedback to development, mobility planning, utilization to new business, and the like) based on the data stored in the mobility server 40A. In this case, the analysis device 50A is used, for example, by a business operator that develops and analyzes the vehicle V

Specifically, the analysis device 50A includes an HMI unit 51, a communication unit 52, a data input and output unit 53, an analysis storage unit 54A, and a processing unit 55. The HMI unit 51, the communication unit 52, the data input and output unit 53, the analysis storage unit 54A, and the processing unit 55 are communicably connected to each other.

The HMI unit 51 is a device that performs various inputs and outputs in the analysis device 50A. Similarly to the HMI unit 12 described above, the HMI unit 51 includes an operation input device that performs various inputs to the analysis device 50A and an information output device that performs various outputs from the analysis device 50A.

The communication unit 52 is a communication module capable of communicating with devices other than the analysis device 50A. The communication unit 52 has substantially the same configuration as the communication unit 21 described above, is communicably connected to the network NW regardless of wireless communication or wired communication, and communicates with other devices (for example, data processing device 20, mobility server 40A, medical server 40B, determination device 50B, user terminal 60, and medical institution terminal 70) via the network NW.

The data input and output unit 53 is a data input and output device that inputs and outputs data (information) to and from the analysis device 50A. The data input and output unit 53 has substantially the same configuration as the data input and output unit 14 described above, and is implemented by a recording medium interface or the like.

The analysis storage unit 54A is a storage circuit that stores various kinds of data. The analysis storage unit 54Ahas substantially the same configuration as the in-vehicle storage unit 15 described above, and is implemented by, for example, an HDD, an SSD, an optical disk, and a semiconductor memory. The analysis storage unit 54A stores, for example, a program that enables units of the analysis device 50A to implement various functions. The analysis storage unit 54A stores various kinds of data necessary for various kinds of processes in the processing unit 55, and the processing unit 55 or the like reads the various kinds of data as necessary.

The processing unit 55 is a processing circuit that integrally controls the analysis device 50A and implements various processing functions of the analysis device 50A. The processing unit 55 has substantially the same configuration as the processing unit 16 described above, and is implemented by, for example, a processor, a RAM, and a ROM. The processing unit 55 implements processing functions, for example, by reading and executing the program stored in the analysis storage unit 54A. For example, the processing unit 55 is capable of executing a process of operating the HMI unit 51, a process of performing data communication via the communication unit 52, and the like.

The processing unit 55 according to the present embodiment includes an HMI processing unit 55a, a data processing unit 55b, a communication processing unit 55c, and an analysis processing unit 55d in order to implement the various processing functions of the analysis device 50A. The processing unit 55 implements processing functions of the HMI processing unit 55a, the data processing unit 55b, the communication processing unit 55c, and the analysis processing unit 55d by reading and executing the program stored in the analysis storage unit 54A, for example.

Similarly to the HMI processing unit 16b described above, the HMI processing unit 55a is a unit having a function capable of executing a process of controlling the HMI unit 51 and operating the HMI unit 51. The HMI processing unit 55a is capable of executing a process of controlling an operation input device constituting the HMI unit 51 and inputting an operation via the operation input device. The HMI processing unit 55a is capable of executing a process of controlling the information output device constituting the HMI unit 51 and outputting information via the information output device.

Similarly to the data processing unit 44b described above, the data processing unit 55b is a unit having a function capable of executing a process related to various kinds of data in the analysis device 50A. The data processing unit 55b is capable of executing a process of storing data received via the communication unit 52 in the analysis storage unit 54A. The data processing unit 55b is capable of executing a process of reading data transmitted via the communication unit 52 from the analysis storage unit 54A. The data processing unit 55b is also capable of executing a process of storing data input via the data input and output unit 53 in the analysis storage unit 54A and a process of reading data output via the data input and output unit 53 from the analysis storage unit 54A. The data processing unit 55b is also capable of executing a process of storing registered data in the analysis storage unit 54A in accordance with an operation on the operation input device constituting the HMI unit 51, and a process of reading the data from the analysis storage unit 54A.

Similarly to the communication processing unit 44a described above, the communication processing unit 55c is a unit having a function capable of executing a process of controlling the communication unit 52 and performing data communication. The communication processing unit 55c is capable of executing a process of transmitting and receiving data to and from other devices such as the data processing device 20, the mobility server 40A, the medical server 40B, the determination device 50B, the user terminal 60, and the medical institution terminal 70 via the communication unit 52.

The analysis processing unit 55d is a unit having a function capable of executing a process of analyzing data stored in the mobility server 40A. The analysis processing unit 55d executes a process of acquiring the vehicle data D1 and the like from the mobility server 40A in accordance with an operation on the operation input device constituting the HMI unit 51. In response to acquiring the data necessary for executing the requested analysis in the analysis device 50A from the vehicle data D1 and the like stored in the mobility server 40A, the analysis processing unit 55d performs analysis based on the data. The data processing unit 55b of the analysis device 50A executes a process of storing the analysis result obtained by the analysis processing unit 55d as analysis data D9 in the analysis storage unit 54A. The HMI processing unit 55a of the analysis device 50A is capable of executing a process of controlling the information output device constituting the HMI unit 51 and outputting the analysis data D9 related to an analysis result obtained by the analysis processing unit 55d via the information output device.

The analysis storage unit 54A of the analysis device 50A includes a vehicle data storage unit 54a, a user data storage unit 54b, a determination result data storage unit 54d, and a medical information data storage unit 54e in terms of functional concept. When the analysis device 50A performs various analyses based on the data stored in the mobility server 40A, the analysis device 50A further includes an analysis data storage unit 54h.

The vehicle data storage unit 54a is a storage area for storing the vehicle data D1 in the analysis storage unit 54A. The vehicle data storage unit 54a stores the vehicle data D1 acquired from the mobility server 40A. The vehicle data D1 acquired from the medical server 40B via the determination device 50B may be stored. The vehicle data storage unit 54a stores the vehicle data D1 including data related to the vehicle identification information D10, the DCM information D11, the vehicle basic information D12, the traveling information D13, and the like received from the data processing device 20.

The user data storage unit 54b is a storage area for storing the user data D2 in the analysis storage unit 54A. For example, the user data storage unit 54b acquires, from the mobility server 40A, the data related to the user basic information D20, the user attribute information D21, the agreement history information D22, the service setting information D23, the contract package information D24, the favorite information D25, and the like registered via the in-vehicle device 10, the user terminal 60, or the like, or automatically generated by the processing unit 44 of the mobility server 40A, and stores the data as the user data D2.

The determination result data storage unit 54d is a storage area for storing the determination result data D5 in the analysis storage unit 54A. The determination result data storage unit 54d stores the determination result data D5 generated in the determination device 50B. The determination result data storage unit 54d stores, for example, the determination result data D5 including data related to the determination result information D51 and the like.

The medical information data storage unit 54e is a storage area for storing the medical information data D6 in the analysis storage unit 54A. The medical information data storage unit 54e stores the medical information data D6 received from the medical institution terminal 70. The medical information data storage unit 54e stores, for example, the medical information data D6 including data related to the determination information D61, the treatment program information D62, the consultation recommendation information D63, and the like.

The analysis data storage unit 54h is a storage area for storing the analysis data D9. The analysis data storage unit 54h stores the analysis data D9 including data related to the analysis result obtained by the analysis processing unit 55d and the like.

The processing unit 55 of the analysis device 50A includes the HMI processing unit 55a, the data processing unit 55b, the communication processing unit 55c, and the analysis processing unit 55d described above in terms of functional concept.

The HMI processing unit 55a of the analysis device 50A is capable of executing a process of controlling the information output device constituting the HMI unit 51, and outputting the determination result data D5 received from the determination device 50B or the medical information data D6 received from the medical institution terminal 70 via the information output device. The data processing unit 55b of the analysis device 50A is capable of executing a process of storing the determination result data D5 received from the determination device 50B in the determination result data storage unit 54d.

The communication processing unit 55c of the analysis device 50A is configured to execute a process of acquiring the user data D2 from the mobility server 40A via the communication unit 52. The communication processing unit 55c of the analysis device 50A is configured to execute a process of acquiring the vehicle data D1 from the medical server 40B and the determination device 50B via the communication unit 52. The communication processing unit 55c is configured to store the acquired vehicle data D1 in the vehicle data storage unit 54a, and store the acquired user data D2 in the vehicle data storage unit 54a. The communication processing unit 55c is configured to execute a process of acquiring the medical information data D6 from the medical institution terminal 70 via the communication unit 52. The communication processing unit 55c is configured to store the acquired medical information data D6 in the medical information data storage unit 54e. The communication processing unit 55c is configured to transmit the user data D2 and the determination result data D5 in association with each other to the medical institution terminal 70.

The communication processing unit 55c of the analysis device 50A is capable of executing a process of transmitting various commands and requests to the mobility server 40A via the communication unit 52. The communication processing unit 55c of the analysis device 50A is capable of executing a process of receiving data related to various kinds of information from the medical server 40B, the determination device 50B, and the medical institution terminal 70 via the communication unit 52.

The analysis processing unit 55d executes a process of acquiring the vehicle data D1 and the like from the mobility server 40A in accordance with an operation on the operation input device constituting the HMI unit 51. The analysis processing unit 55d receives data necessary for executing the requested analysis in the analysis device 50A from the vehicle data D1 and the like stored in the mobility server 40A, and analyzes the data based on the data. The data processing unit 55b of the analysis device 50A is capable of executing a process of storing the analysis result obtained by the analysis processing unit 55d as the analysis data D9 in the analysis storage unit 54A. The HMI processing unit 55a of the analysis device 50A is capable of executing a process of controlling the information output device constituting the HMI unit 51 and outputting the analysis data D9 such as an analysis result obtained by the analysis processing unit 55d via the information output device.

### <Basic Configuration of Medical Server>

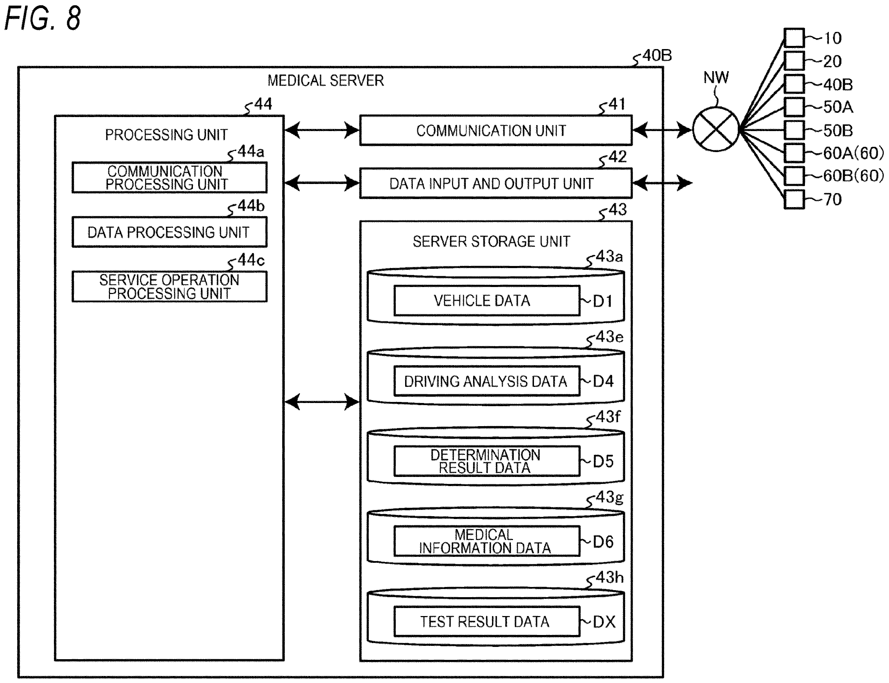

As described above, the medical server 40B shown in FIG. 8 is a server that mutually cooperates with the mobility server 40A and manages data mainly used for the service provided to the user of the vehicle V

A basic configuration of the medical server 40B is substantially the same as that of the mobility server 40A. The medical server 40B includes the communication unit 41, the data input and output unit 42, the server storage unit 43, and the processing unit 44. The communication unit 41, the data input and output unit 42, the server storage unit 43, and the processing unit 44 are communicably connected to each other.

The communication unit 41 of the medical server 40B is a communication module capable of communicating with devices other than the medical server 40B. The communication unit 41 has substantially the same configuration as the communication unit 21 described above, is communicably connected to the network NW regardless of wireless communication or wired communication, and communicates with other devices (for example, data processing device 20, analysis device 50A, determination device 50B, and user terminal 60) via the network NW.

The data input and output unit 42 of the medical server 40B is a data input and output device that inputs and outputs data (information) to and from the medical server 40B. The data input and output unit 42 has substantially the same configuration as the data input and output unit 14 described above, and is implemented by a recording medium interface or the like.

The server storage unit 43 of the medical server 40B has substantially the same configuration as the server storage unit 43 of the mobility server 40A. Similarly to the server storage unit 43 of the mobility server 40A, the server storage unit 43 of the medical server 40B stores various kinds of data necessary for various kinds of processes in the processing unit 44, and the processing unit 44 or the like reads the various kinds of data as necessary.

Here, the server storage unit 43 of the medical server 40B includes the vehicle data storage unit 43a, a driving analysis data storage unit 43e, a determination result data storage unit 43f, a medical information data storage unit 43g, and a test result data storage unit 43h in terms of functional concept.

The vehicle data storage unit 43a has substantially the same configuration as the vehicle data storage unit 43a of the mobility server 40A described above. The vehicle data storage unit 43a stores the vehicle data D1 transmitted from the in-vehicle device 10. The vehicle data storage unit 43a stores, for example, the vehicle data D1 including data related to the vehicle identification information D10, the DCM information D11, the vehicle basic information D12, the traveling information D13, and the like.

The driving analysis data storage unit 43e is a storage area for storing the driving analysis data D4 in the server storage unit 43. The driving analysis data storage unit 43e stores the driving analysis data D4 transmitted from the determination device 50B to the medical server 40B. The driving analysis data storage unit 43e stores, for example, the driving analysis data D4 including data related to the driving analysis information D41 and the like.

The determination result data storage unit 43f is a storage area for storing the determination result data D5 in the server storage unit 43. The determination result data storage unit 43f is a storage area for storing the determination result data D5 transmitted from the determination device 50B to the medical server 40B. The determination result data storage unit 43f stores the determination result data D5 including data related to the determination result information D51.

The medical information data storage unit 43g is a storage area for storing the medical information data D6 in the server storage unit 43. The medical information data storage unit 43g is a storage area for storing the medical information data D6 transmitted from the medical institution terminal 70 to the medical server 40B via the determination device 50B. The medical information data storage unit 43g stores, for example, the medical information data D6 including data related to the determination information D61, the treatment program information D62, the consultation recommendation information D63, and the like.

The test result data storage unit 43h is a storage area for storing the test result data DX in the server storage unit 43. The test result data storage unit 43h stores, for example, the test result data DX transmitted from the determination device 50B to the medical server 40B. The test result data storage unit 43h stores the test result data DX including data related to the test result information DX1.

The processing unit 44 is a processing circuit that integrally controls the medical server 40B and implements various processing functions of the medical server 40B. The processing unit 44 has substantially the same configuration as the processing unit 44 of the mobility server 40A. For example, the processing unit 44 is capable of executing a process of performing data communication via the communication unit 41, a process of calculating various kinds of data, a process of providing various services to the user, and the like.

The processing unit 44 according to the present embodiment includes a communication processing unit 44a, a data processing unit 44b, and a service operation processing unit 44c in terms of functional concept in order to implement the various processing functions described above. The processing unit 44 implements processing functions of the communication processing unit 44a, the data processing unit 44b, and the service operation processing unit 44c, for example, by reading and executing the program stored in the server storage unit 43.

The communication processing unit 44a is a unit having a function capable of executing a process of controlling the communication unit 41 and performing data communication. The communication processing unit 44a is capable of executing a process of transmitting and receiving data to and from other devices such as the data processing device 20 and the user terminal 60 via the communication unit 41.

The communication processing unit 44a is capable of executing a process of receiving the vehicle data D1 transmitted from the data processing device 20 to the medical server 40B via the communication unit 41. The communication processing unit 44a is capable of executing a process of receiving various commands and requests from the data processing device 20, the analysis device 50A, the determination device 50B, and the like via the communication unit 41.

Similarly to the mobility server 40A, the data processing unit 44b is a unit having a function capable of executing a process related to various kinds of data in the medical server 40B. In the medical server 40B, the data processing unit 44b is capable of executing a process of storing data received via the communication unit 41 in the server storage unit 43. In the medical server 40B, the data processing unit 44b is capable of executing a process of reading data transmitted via the communication unit 41 from the server storage unit 43. In the medical server 40B, the data processing unit 44b is also capable of executing a process of storing data input via the data input and output unit 42 in the server storage unit 43 and a process of reading data output via the data input and output unit 42 from the server storage unit 43.

More specifically, for example, the data processing unit 44b of the medical server 40B is capable of executing a process of storing the vehicle data D 1 transmitted from the in-vehicle device 10 via the data processing device 20 in the vehicle data storage unit 43a. The data processing unit 44b of the medical server 40B is capable of executing a process of storing the driving analysis data D4 and the determination result data D5 transmitted from the determination device 50B in the driving analysis data storage unit 43e and the determination result data storage unit 43f. The data processing unit 44b of the medical server 40B is capable of executing a process of storing the medical information data D6 transmitted from the analysis device 50A in the medical information data storage unit 43g. The data processing unit 44b is capable of executing a process of reading the vehicle data D 1 from the vehicle data storage unit 43a in response to a request. The data processing unit 44b is capable of executing a process of reading the determination result data D5 from the determination result data storage unit 43f in response to a request. The data processing unit 44b is capable of executing a process of reading the medical information data D6 from the medical information data storage unit 43g in response to a request.

As described above, when respectively storing the vehicle data D1, the driving analysis data D4, the determination result data D5, and the medical information data D6 in the vehicle data storage unit 43a, the driving analysis data storage unit 43e, the determination result data storage unit 43f, and the medical information data storage unit 43g, the data processing unit 44b of the medical server 40B is capable of executing a process of associating the vehicle data D1, the driving analysis data D4, the determination result data D5, and the medical information data D6 with each other. For example, the data processing unit 44b associates the vehicle data D1 stored in the vehicle data storage unit 43a, the driving analysis data D4 stored in the driving analysis data storage unit 43e, the determination result data D5 stored in the determination result data storage unit 43f, and the medical information data D6 stored in the medical information data storage unit 43g with each other via the vehicle identification information D10 to form a data set. That is, the vehicle data D1 stored in the vehicle data storage unit 43a, the driving analysis data D4 stored in the driving analysis data storage unit 43e, and the determination result data D5 stored in the determination result data storage unit 43f are stored by the data processing unit 44b in association with the vehicle identification information D 10, and the server storage unit 43 of the medical server 40B stores the above data in a database as a data set.

The service operation processing unit 44c is a unit having a function capable of executing a process related to provision of a service based on commands and requests received from the data processing device 20, the analysis device 50A, the determination device 50B, and the like, and based on data or the like stored in the server storage unit 43. For example, in the medical server 40B, the service operation processing unit 44c transmits the vehicle data D1 to the determination device 50B based on a command or a request received from the determination device 50B.

### <Basic Configuration of Determination Device>

The determination device 50B shown in FIG. 8 acquires the vehicle data D1 from the medical server 40B, determines the presence or absence, the content, and the like of the abnormal indication of the user based on the acquired vehicle data D1, and transmits the determination result data D5 including information related to the determination result to the medical server 40B. If the determination device 50B determines that there is an abnormal indication in the user, the determination device 50B transmits the determination result data D5 to the medical institution terminal 70 via the analysis device 50A. The determination device 50B is configured to receive access from the user terminal 60. Accordingly, the user can access the cognitive function determination data D0 stored in the determination device 50B from the user terminal 60, and can take the cognitive function test in the user terminal 60. When the cognitive function test is performed by the user, the determination device 50B transmits the test result data DX including information related to the result of the cognitive function test to the medical server 40B. Here, the determination device 50B transmits the test result data DX in a manner of being able to be stored in association with other data such as the vehicle data D1 in the medical server 40B. Specifically, when the user performs the cognitive function test, the determination device 50B acquires the vehicle identification information D10 and the like of the user from the user terminal 60, and transmits the test result data DX, the acquired vehicle identification information D10, and the like in association with each other to the medical server 40B. Accordingly, the medical server 40B can store the test result data DX, the vehicle data D1, and the like in association with each other based on the vehicle identification information D10 and the like associated with the test result data DX. The determination device 50B is not limited to transmitting the test result data DX in a manner of being able to be stored in association with the vehicle data D1, but may transmit the test result data DX to the medical server 40B in a manner of being able to be stored in association with the user data D2.

Specifically, the determination device 50B includes the HMI unit 51, the communication unit 52, the data input and output unit 53, a determination storage unit 54B, and the processing unit 55. The HMI unit 51, the communication unit 52, the data input and output unit 53, the determination storage unit 54B, and the processing unit 55 are communicably connected to each other.

The HMI unit 51 is a device that performs various inputs and outputs in the determination device 50B. Similarly to the HMI unit 12 described above, the HMI unit 51 includes an operation input device that performs various inputs to the determination device 50B and an information output device that performs various outputs from the determination device 50B.

The communication unit 52 is a communication module capable of communicating with devices other than the determination device 50B. The communication unit 52 has substantially the same configuration as the communication unit 21 described above, is communicably connected to the network NW regardless of wireless communication or wired communication, and communicates with other devices (for example, data processing device 20, mobility server 40A, medical server 40B, analysis device 50A, user terminal 60, and medical institution terminal 70) via the network NW.

The data input and output unit 53 is a data input and output device that inputs and outputs data (information) to and from the determination device 50B. The data input and output unit 53 has substantially the same configuration as the data input and output unit 14 described above, and is implemented by a recording medium interface or the like.

The determination storage unit 54B is a storage circuit that stores various kinds of data. The determination storage unit 54B has substantially the same configuration as the in-vehicle storage unit 15 described above, and is implemented by, for example, an HDD, an SSD, an optical disk, and a semiconductor memory. The determination storage unit 54B stores, for example, programs that enable units of the determination device 50B to implement various functions. The determination storage unit 54B stores various kinds of data necessary for various kinds of processes in the processing unit 55, and the processing unit 55 or the like reads the various kinds of data as necessary.

The processing unit 55 is a processing circuit that integrally controls the determination device 50B and implements various processing functions of the determination device 50B. The processing unit 55 has substantially the same configuration as the processing unit 16 described above, and is implemented by, for example, a processor, a RAM, and a ROM. The processing unit 55 implements processing functions, for example, by reading and executing the programs stored in the determination storage unit 54B. For example, the processing unit 55 is capable of executing a process of operating the HMI unit 51, a process of performing data communication via the communication unit 52, and the like.

The processing unit 55 according to the present embodiment includes the HMI processing unit 55a, the data processing unit 55b, and the communication processing unit 55c in order to implement the various processing functions of the determination device 50B. The processing unit 55 implements processing functions of the HMI processing unit 55a, the data processing unit 55b, and the communication processing unit 55c by reading and executing the program stored in the determination storage unit 54B, for example.

Similarly to the HMI processing unit 16b described above, the HMI processing unit 55a is a unit having a function capable of executing a process of controlling the HMI unit 51 and operating the HMI unit 51. The HMI processing unit 55a is capable of executing a process of controlling an operation input device constituting the HMI unit 51 and inputting an operation via the operation input device. The HMI processing unit 55a is capable of executing a process of controlling the information output device constituting the HMI unit 51 and outputting information via the information output device.

Similarly to the data processing unit 44b described above, the data processing unit 55b is a unit having a function capable of executing a process related to various kinds of data in the determination device 50B. The data processing unit 55b is capable of executing a process of storing data received via the communication unit 52 in the determination storage unit 54B. The data processing unit 55b is capable of executing a process of reading data transmitted via the communication unit 52 from the determination storage unit 54B. The data processing unit 55b is also capable of executing a process of storing, in the determination storage unit 54B, data input via the data input and output unit 53 and a process of reading data output via the data input and output unit 53 from the determination storage unit 54B. The data processing unit 55b is also capable of executing a process of storing, in the determination storage unit 54B, data registered in accordance with an operation on the operation input device constituting the HMI unit 51, and a process of reading the data from the determination storage unit 54B.

Similarly to the communication processing unit 44a described above, the communication processing unit 55c is a unit having a function capable of executing a process of controlling the communication unit 52 and performing data communication. The communication processing unit 55c is capable of executing a process of transmitting and receiving data to and from other devices such as the data processing device 20, the mobility server 40A, the medical server 40B, the user terminal 60, and the medical institution terminal 70 via the communication unit 52.

The determination processing unit 55e is a unit that analyzes a driving state of the user from the vehicle data D1 stored in the determination storage unit 54B according to a predetermined cycle or an operation on the operation input device constituting the HMI unit 51, and performs the determination related to the abnormal indication of the user based on the driving analysis data D4 including the information related to the analysis result. Here, the determination processing unit 55e reads the vehicle data D1 from the determination storage unit 54B, analyzes the driving state of the user from the read vehicle data D1, performs a process of extracting an abnormal driving behavior of the user in the vehicle V based on the analysis and the like, and generates the driving analysis data D4. Then, the determination processing unit 55e reads determination reference data D8 from the determination storage unit 54B, determines the presence or absence, the content, and the like of the abnormal indication of the user based on the read determination reference data D8 and the driving analysis data D4, and generates the determination result data D5 including the information related to the analysis result.

The HMI processing unit 55a of the determination device 50B is capable of executing a process of controlling the information output device constituting the HMI unit 51, and outputting the determination result data D5 generated by the determination processing unit 55e or the medical information data D6 received from the medical institution terminal 70 via the information output device. The data processing unit 55b of the determination device 50B is capable of executing a process of storing the determination result data D5 generated by the determination processing unit 55e in the determination result data storage unit 54d.

The determination storage unit 54B of the determination device 50B includes the vehicle data storage unit 54a, a driving analysis data storage unit 54c, the determination result data storage unit 54d, a determination reference data storage unit 54f, and a cognitive function determination data storage unit 54g in terms of functional concept.

The vehicle data storage unit 54a is a storage area for storing the vehicle data D 1 acquired from the medical server 40B in the determination storage unit 54B. The vehicle data storage unit 54a stores the vehicle data D 1 including data related to the vehicle identification information D10, the DCM information D11, the vehicle basic information D12, the traveling information D13, and the like received from the data processing device 20.

The driving analysis data storage unit 54c is a storage area for storing the driving analysis data D4 in the determination storage unit 54B. The driving analysis data storage unit 54c stores the driving analysis data D4 generated in the determination device 50B. The driving analysis data storage unit 54c stores, for example, the driving analysis data D4 including data related to the driving analysis information D41 and the like.

The determination result data storage unit 54d is a storage area for storing the determination result data D5 in the determination storage unit 54B. The determination result data storage unit 54d stores the determination result data D5 generated in the determination device 50B. The determination result data storage unit 54d stores, for example, the determination result data D5 including data related to the determination result information D51 and the like.

The determination reference data storage unit 54f is a storage area for storing, in the determination storage unit 54B, the determination reference data D8 which is data related to a reference for determining the presence or absence, the content, and the like of the abnormal indication of the user based on the driving analysis data D4. The determination reference data D8 includes information related to a reference created based on the correlation relation between the driving operation of the user in the vehicle V and the cognitive function state of the user as a reference for determining the presence or absence, the content, and the like of the abnormal indication of the user. The determination reference data D8 includes, for example, information for determining that the user driving the vehicle V has an indication of deterioration in cognitive function in a case where a day on which an occurrence frequency of an abnormal driving behavior performed by a person having an abnormal cognitive function exceeds a predetermined number of times continues for a predetermined period. Here, the "predetermined number of times" and the "predetermined period" are set to the number of times and the period that can be distinguished from a case where an abnormal driving behavior temporarily occurs, for example, a case where the user is forced to suddenly operate the steering wheel or suddenly brake due to a surrounding situation of the vehicle V or a case where the user intentionally takes an abnormal driving behavior for a short time.

The cognitive function determination data storage unit 54g is a storage area for storing the cognitive function determination data D0 in the determination storage unit 54B. The cognitive function determination data storage unit 54g stores the cognitive function determination data D0 as information for the determination device 50B to execute the cognitive function test.

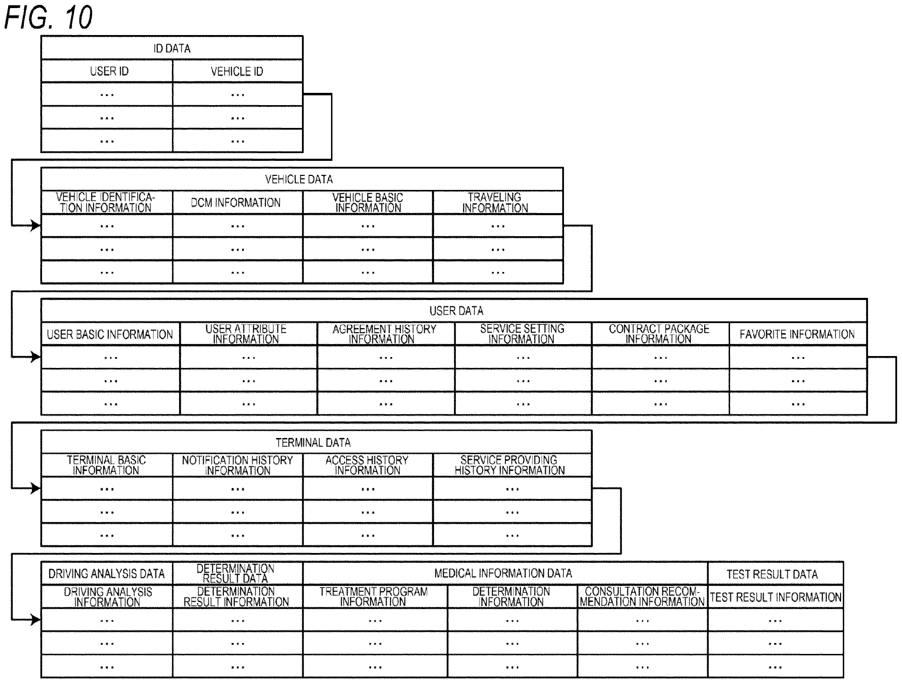

FIG. 10 is an example of a database stored in the mobility server 40A and the medical server 40B that constitute the storage unit of the abnormality determination system 1. In the present embodiment, the mobility server 40A and the medical server 40B cooperate with each other to store the vehicle data D1, the user data D2, the terminal data D3, the driving analysis data D4, the determination result data D5, the medical information data D6, the test result data DX, and the ID data D7 in association with each other as a database in a table format.

### <Basic Configuration of User Terminal>

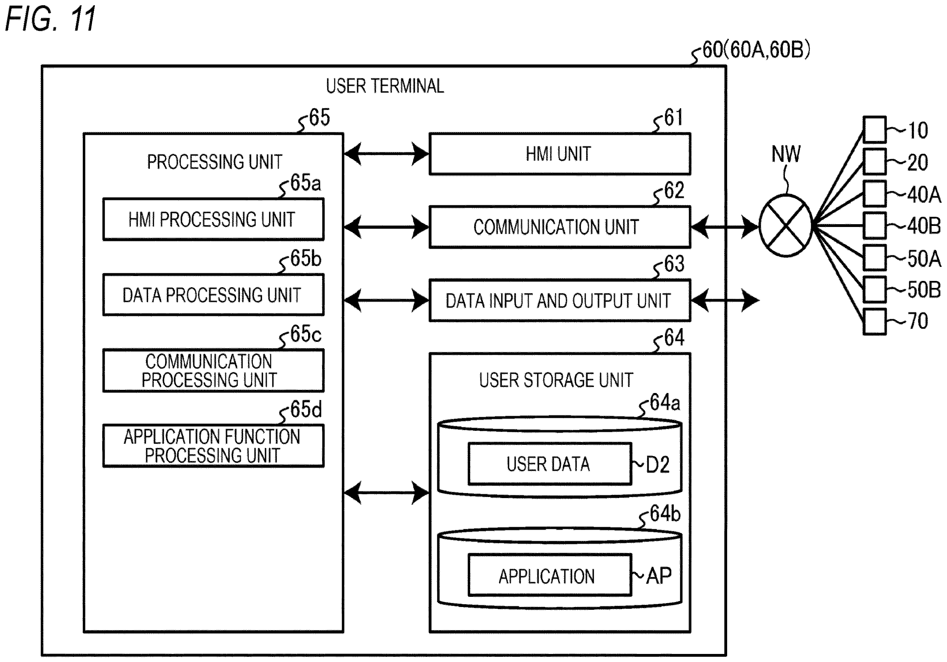

As described above, the user terminal 60 shown in FIG. 11 is a terminal device configured to receive the medical information notification from the analysis device 50A. The user terminal 60 includes the first user terminal 60A that is mainly owned and used by the user, and the second user terminal 60B that is mainly owned and used by a family of the user, a related person, or the like. Here, there is no substantial difference in configuration between the first user terminal 60A and the second user terminal 60B. The user terminal 60 may be configured to receive various services (for example, contract management, parking position search, vehicle remote control, emergency notification support, trouble support, security notification, and driving history evaluation) based on data stored in the mobility server 40A.

Specifically, the user terminal 60 includes an HMI unit 61, a communication unit 62, a data input and output unit 63, a user storage unit 64, and a processing unit 65. The HMI unit 61, the communication unit 62, the data input and output unit 63, the user storage unit 64, and the processing unit 65 are communicably connected to each other.

The HMI unit 61 is a device that performs various inputs and outputs in the user terminal 60. Similarly to the HMI unit 12 described above, the HMI unit 61 includes an operation input device serving as an input unit that performs various inputs to the user terminal 60, and an information output device serving as an output unit that performs various outputs from the user terminal 60.

The communication unit 62 is a communication module capable of communicating with other devices. The communication unit 62 has substantially the same configuration as the communication unit 41 described above, is communicably connected to the network NW regardless of wireless communication or wired communication, and communicates with other devices (for example, data processing device 20, mobility server 40A, medical server 40B, analysis device 50A, determination device 50B, and medical institution terminal 70) via the network NW.

The data input and output unit 63 is a data input and output device that inputs and outputs data (information) to and from the user terminal 60. The data input and output unit 63 has substantially the same configuration as the data input and output unit 14 described above, and is implemented by a recording medium interface or the like.

The user storage unit 64 is a storage circuit that stores various kinds of data. The user storage unit 64 has substantially the same configuration as the in-vehicle storage unit 15 described above, and is implemented by, for example, an HDD, an SSD, an optical disk, and a semiconductor memory. The user storage unit 64 stores, for example, a program that enables units of the user terminal 60 to implement various functions. The user storage unit 64 stores various kinds of data necessary for various kinds of processes in the processing unit 65, and the processing unit 65 or the like reads the various kinds of data as necessary.

The processing unit 65 is a processing circuit that integrally controls the user terminal 60 and implements various processing functions of the user terminal 60. The processing unit 65 has substantially the same configuration as the processing unit 16 described above, and is implemented by, for example, a processor, a RAM, and a ROM. The processing unit 65 implements processing functions by reading and executing the program stored in the user storage unit 64, for example. For example, the processing unit 65 is capable of executing a process of operating the HMI unit 61, a process of performing data communication via the communication unit 62, and the like.

The processing unit 65 according to the present embodiment includes an HMI processing unit 65a, a data processing unit 65b, and a communication processing unit 65c in order to implement various processing functions of the user terminal 60. The processing unit 65 implements processing functions of the HMI processing unit 65a, the data processing unit 65b, and the communication processing unit 65c, for example, by reading and executing the programs stored in the user storage unit 64.

Similarly to the HMI processing unit 16b described above, the HMI processing unit 65a is a unit having a function capable of executing a process of controlling the HMI unit 61 and operating the HMI unit 61. The HMI processing unit 65a is capable of executing a process of controlling the operation input device constituting the HMI unit 61 and inputting the operation via the operation input device. The HMI processing unit 65a is capable of executing a process of controlling the information output device constituting the HMI unit 61 and outputting information via the information output device.

Similarly to the data processing unit 44b described above, the data processing unit 65b is a unit having a function capable of executing a process related to various kinds of data in the user terminal 60. The data processing unit 65b is capable of executing a process of storing data received via the communication unit 62 in the user storage unit 64. The data processing unit 65b is capable of executing a process of reading data transmitted via the communication unit 62 from the user storage unit 64. The data processing unit 65b is also capable of executing a process of storing data input via the data input and output unit 63 in the user storage unit 64 and a process of reading data output via the data input and output unit 63 from the user storage unit 64. The data processing unit 65b is also capable of executing a process of storing, in the user storage unit 64, data registered in accordance with an operation on the operation input device constituting the HMI unit 61 and a process of reading the data from the user storage unit 64.

Similarly to the communication processing unit 44a described above, the communication processing unit 65c is a unit having a function capable of executing a process of controlling the communication unit 62 and performing data communication. The communication processing unit 65c is capable of executing a process of transmitting and receiving data to and from other devices such as the data processing device 20, the mobility server 40A, the medical server 40B, the analysis device 50A, the determination device 50B, and the medical institution terminal 70 via the communication unit 62.

The user storage unit 64 of the user terminal 60 includes a user data storage unit 64a and an application storage unit 64b in terms of functional concept.

The user data storage unit 64a is a storage area for storing the user data D2 to be transmitted to the mobility server 40A in the user storage unit 64. The user data storage unit 64a stores, for example, the user data D2 including data related to the user basic information D20, the service setting information D23, the favorite information D25, and the like registered in accordance with an operation of the user on the operation input device constituting the HMI unit 61 of the first user terminal 60A.

The application storage unit 64b is a storage area for storing an application program AP used in the user terminal 60 in the user storage unit 64. The application program AP stored in the application storage unit 64b is executed in the user terminal 60 to receive the notification of the medical information transmitted from the analysis device 50A and output the content of the medical information notification to the output unit of the HMI unit 61. Specifically, the HMI unit 61 displays, for example, a determination result related to the cognitive function of the user, the treatment program, and information related to a notification of the consultation recommendation on the display unit. Here, the output unit of the HMI unit 61 includes a display unit having a touch panel function capable of inputting and outputting an operation by the user. In the present embodiment, the notification of the consultation recommendation includes presentation of a behavior recommended to the user. When there is the notification of the consultation recommendation, the output unit of the HMI unit 61 presents, to the user, a behavior recommended to the user. Specifically, in response to receiving the notification of the consultation recommendation, the user terminal 60 executes the application program AP, and displays the behavior recommended to the user according to the content of the medical information notification on the display unit of the HMI unit 61. According to this configuration, the behavior recommended to the user is presented to the user. When the input unit of the HMI unit 61 is operated and an instruction to execute the behavior recommended to the user is received, the user terminal 60 can operate the application program AP to execute the behavior recommended to the user. The application program AP is not limited to one that executes a process corresponding to the notification of the consultation recommendation, and may be capable of executing application functions corresponding to various services linked with the mobility server 40A.

The application program AP enables the user to take the cognitive function test by accessing the cognitive function determination data D0 stored in the determination device 50B from the user terminal 60 by the user. In this case, the HMI unit 61 outputs the content of the cognitive function test to the display unit capable of receiving the input operation by the user, and the user can respond to the cognitive function test by performing the input operation on the content output to the display unit. The application program AP is not limited to the execution of the cognitive function test prepared in the determination device 50B, and may be capable of executing application functions corresponding to various services linked with the mobility server 40A.

The processing unit 65 of the user terminal 60 includes the HMI processing unit 65a, the data processing unit 65b, the communication processing unit 65c, and an application function processing unit 65d in terms of functional concept.

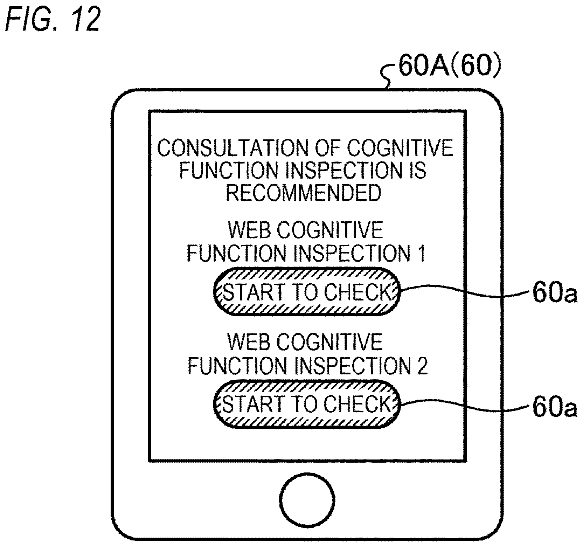

The HMI processing unit 65a of the user terminal 60 is capable of executing a process of controlling the display unit constituting the HMI unit 61, and displaying information and data related to various services executed by the application program AP on the display unit. Each of FIGS. 12 to 14 shows an example of the behavior recommended to the user presented on the display unit when the user terminal 60 displays the notification of the consultation recommendation included in the medical information notification on the display unit of the HMI unit 61.

For example, as shown in FIG. 12, the display unit of the HMI unit 61 displays a message " consultation of cognitive function inspection is recommended" as the behavior recommended to the user, and displays an icon 60a for starting the cognitive function inspection as the input unit of the HMI unit 61 in order for the user to execute the behavior. The user can consult the cognitive function inspection by touching this icon 60a. A plurality of behaviors recommended to the user may be displayed on the display unit of the HMI unit 61. In the example shown in FIG. 12, two kinds of WEB cognitive function inspections (WEB cognitive function inspection 1 and WEB cognitive function inspection 2) having different contents are selectably displayed as the cognitive function test for the user. The user can take the cognitive function test of the determination device 50B by selecting the WEB cognitive function inspections.

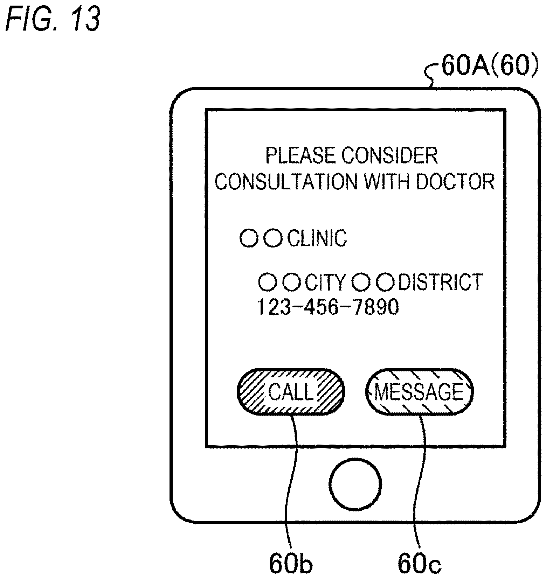

For example, as shown in FIG. 13, the display unit of the HMI unit 61 may display a message "please consider consultation with doctor" as the behavior recommended to the user, and display a call icon 60b that allows the user to make a call to the doctor, and a send icon 60c that allows the user to send a message to the doctor. In this case, when the user terminal 60 can make a phone call to the doctor when receiving a touch operation on the call icon 60b from the user. The user terminal 60 can send a message to the doctor in response to receiving a touch operation on the send icon 60c from the user

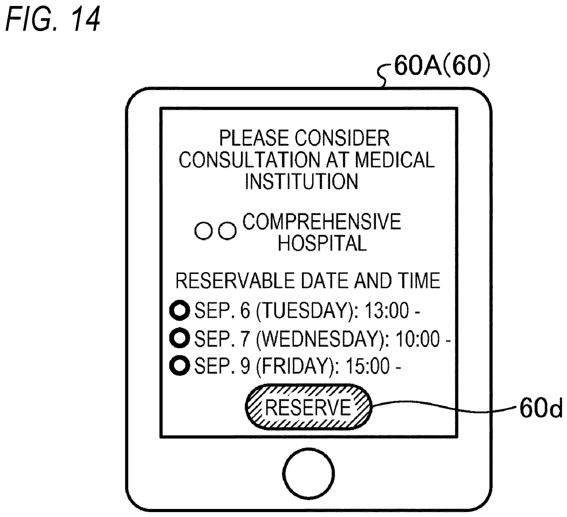

For example, as shown in FIG. 14, the display unit of the HMI unit 61 may display a massage "please consider consultation at medical institution" as the behavior recommended to the user, and display a reservation icon 60d for making a reservation for consultation in the medical institution. In this case, the user terminal 60 can make a reservation for consultation in the medical institution in response to receiving a touch operation on the reservation icon 60d from the user.

The first user terminal 60A owned by the user himself or herself and the second user terminal 60B owned by the family of the user, the related person, or the like may be configured to output the same content, or may be configured to output different contents between the content for the user himself or herself and the content for the family, the related person, or the like. For example, when the notification of the consultation recommendation including a treatment program and a determination result related to the cognitive function of the user is issued, the same content may be output to the first user terminal 60A and the second user terminal 60B, and the family of the user, the related person, or the like may also know the cognitive function state of the user. When different contents between the content for the user himself or herself and the content for the family member, the related person, or the like are output, the notification of the consultation recommendation as shown in FIGS. 12 to 14 described above may be issued on the first user terminal 60A as the content for the user himself or herself, and a notification related to a simple contact indicating that the user is notified of the consultation recommendation to the medical institution may be issued on the second user terminal 60B as the content for the family member, the related person, or the like.

The data processing unit 65b of the user terminal 60 is capable of executing a process related to a behavior recommended to the user presented on a display unit of the user terminal 60 in accordance with an operation on the operation input device constituting the HMI unit 61. The data processing unit 65b of the user terminal 60 is capable of executing a process of storing, in the user data storage unit 64a as the user data D2, data related to the user basic information D20, the service setting information D23, the favorite information D25, and the like registered in accordance with an operation on the operation input device constituting the HMI unit 61. The data processing unit 65b of the user terminal 60 is capable of executing a process of reading the user data D2 stored in the user data storage unit 64a in response to a request.

The communication processing unit 65c of the user terminal 60 is capable of executing a process of transmitting the user data D2 stored in the user data storage unit 64a to the mobility server 40A via the communication unit 62. The communication processing unit 65c of the user terminal 60 is capable of executing a process of transmitting various commands and requests to the mobility server 40A or the like via the communication unit 62. The communication processing unit 65c of the user terminal 60 is capable of executing a process of receiving the various commands and requests from the mobility server 40A, the analysis device 50A, and the like via the communication unit 62. The communication processing unit 65c of the user terminal 60 is capable of executing a process of receiving various kinds of information according to a service content and an application function from the mobility server 40A, the analysis device 50A, or the like via the communication unit 62. The data received by the communication processing unit 65c of the user terminal 60 from the mobility server 40A, the analysis device 50A, or the like according to the service content or the application function may include, for example, the vehicle data D 1 and the user data D2 stored in the mobility server 40A, and various kinds of information and data generated based on the above data.

The application function processing unit 65d is a unit having a function capable of executing a process of executing the application program AP stored in the application storage unit 64b. The application function processing unit 65d activates the application program AP stored in the application storage unit 64b in response to, for example, receiving the notification of the medical information from the analysis device 50A or an operation performed on the operation input device constituting the HMI unit 61, and is capable of executing application functions corresponding to various services.

### <Basic Configuration of Medical Institution Terminal>

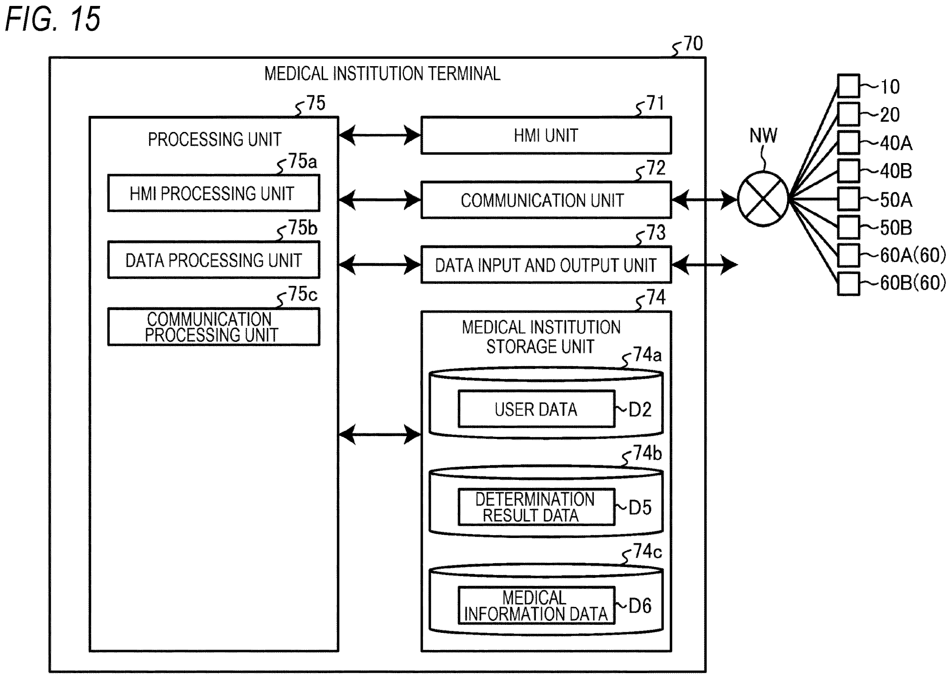

As described above, the medical institution terminal 70 shown in FIG. 15 is configured to communicate with the analysis device 50A, and is a device mainly used by the medical worker such as a doctor. Typically, the medical institution terminal 70 is a terminal device mainly provided at a medical institution. The medical institution terminal 70 is configured to receive the user data D2 and the determination result data D5 transmitted from the analysis device 50A and store the data in the storage unit. The medical institution terminal 70 is configured such that the medical worker such as a doctor can confirm the contents of the user data D2 and the determination result data D5. The medical institution terminal 70 is configured to store, in the storage unit, the medical information data D6 created based on the determination result data D5 and the opinion of the medical worker, and transmit the medical information data D6 to the analysis device 50A. The medical institution terminal 70 may be configured to access a server, a storage terminal, or the like in which diagnosis information such as an electronic medical record is stored. In this case, the medical institution terminal 70 is configured to specify the diagnosis information of the user in the medical institution from the user data D2. When the diagnosis information of the user in the medical institution can be specified from the user data D2, the medical worker can create the medical information data D6 in consideration of the content of the diagnosis information of the user in addition to the determination result data D5. The user data D2 stored in the medical institution terminal 70 includes information that can be collated with diagnosis information of a patient stored in the medical institution. For example, the medical institution terminal 70 collates a personal number or a medical insurance card number included in the user data D2 with a personal number or a medical insurance card number included in the diagnosis information of the user in the medical institution to specify the diagnosis information of the target user. The diagnosis information of the target user may be specified by utilizing information such as a name, an address, a telephone number, an age, and a gender of the user included in the user data D2. The medical institution terminal 70 may transmit the medical information data D6 to a storage device such as a server or a storage terminal provided in the medical institution, and store the medical information data D6 in association with the diagnosis information (electronic medical record or the like) of the user stored in the storage device. The medical institution terminal 70 may be configured to access the medical server 40B. In this case, for example, the medical institution terminal 70 can receive the vehicle data D1 together with the determination result data D5 from the analysis device 50A, utilizes the vehicle identification information D10 included in the received vehicle data D1 to specify the vehicle identification information D10 stored in the medical server 40B, and browse or acquire the test result data DX and the like of the cognitive function test corresponding to the specified vehicle identification information D10. The user can be specified from the vehicle identification information D10 based on a relation between the vehicle data D1 and the user data D2. The medical institution terminal 70 is not limited to specifying the user based on the vehicle data D1, but may be configured to directly specify the user by utilizing the user data D2.

The medical institution terminal 70 includes an HMI unit 71, a communication unit 72, a data input and output unit 73, a medical institution storage unit 74, and a processing unit 75. The HMI unit 71, the communication unit 72, the data input and output unit 73, the medical institution storage unit 74, and the processing unit 75 are communicably connected to each other.

The HMI unit 71 is a device that performs various inputs and outputs in the medical institution terminal 70. Similarly to the HMI unit 12 described above, the HMI unit 71 includes an operation input device that performs various inputs to the medical institution terminal 70 and an information output device that performs various outputs from the medical institution terminal 70.

The communication unit 72 is a communication module capable of communicating with devices other than the medical institution terminal 70. The communication unit 72 has substantially the same configuration as the communication unit 21 described above, is communicably connected to the network NW regardless of wireless communication or wired communication, and communicates with other devices (for example, service providing device 30 (mobility server 40A, medical server 40B, analysis device 50A, and determination device 50B)) via the network NW.

The data input and output unit 73 is a data input and output device that inputs and outputs data (information) to and from the medical institution terminal 70. The medical institution terminal 70 has substantially the same configuration as the data input and output unit 14 described above, and is implemented by a recording medium interface or the like.

The processing unit 75 of the medical institution terminal 70 includes an HMI processing unit 75a, a data processing unit 75b, and a communication processing unit 75c in terms of functional concept.

Similarly to the HMI processing unit 16b described above, the HMI processing unit 75a of the medical institution terminal 70 is a unit having a function capable of executing a process of controlling the HMI unit 71 and operating the HMI unit 71. The HMI processing unit 75a is capable of executing a process of controlling the operation input device constituting the HMI unit 71 and inputting an operation via the operation input device. The HMI processing unit 75a is capable of executing a process of controlling the information output device constituting the HMI unit 71 and outputting information via the information output device.

Similarly to the data processing unit 44b described above, the data processing unit 75b of the medical institution terminal 70 is a unit having a function capable of executing a process related to various kinds of data in the user terminal 60. The data processing unit 75b is capable of executing a process of storing data received via the communication unit 72 in the medical institution storage unit 74. The data processing unit 75b is capable of executing a process of reading the data transmitted via the communication unit 72 from the medical institution storage unit 74. The data processing unit 75b is also capable of executing a process of storing data input via the data input and output unit 73 in the medical institution storage unit 74, and a process of reading data output via the data input and output unit 73 from the medical institution storage unit 74. The data processing unit 75b is also capable of executing a process of storing, in the medical institution storage unit 74, data created according to an operation on the operation input device constituting the HMI unit 51, and a process of reading the data from the medical institution storage unit 74.

The communication processing unit 75c of the medical institution terminal 70 is capable of executing a process of receiving the user data D2 and the determination result data D5 from the analysis device 50A via the communication unit 72. The communication processing unit 75c is capable of executing a process of transmitting the medical information data D6 to the analysis device 50A via the communication unit 72.

Similarly to the communication processing unit 44a described above, the communication processing unit 75c of the medical institution terminal 70 is a unit having a function of executing a process of controlling the communication unit 72 and performing the data communication. The communication processing unit 75c is capable of executing a process of transmitting and receiving data to and from other devices such as the data processing device 20, the analysis device 50A, and the user terminal 60 via the communication unit 72.

The medical institution storage unit 74 includes a user data storage unit 74a, a determination result data storage unit 74b, and a medical information data storage unit 74c in terms of functional concept.

The user data storage unit 74a of the medical institution terminal 70 is a storage area for storing the user data D2 in the medical institution terminal 70. The user data storage unit 74a stores the user data D2 transmitted from the analysis device 50A in association with the determination result data D5. The determination result data storage unit 74b of the medical institution terminal 70 is a storage area for storing the determination result data D5 in the medical institution terminal 70. The determination result data storage unit 74b stores the determination result data D5 transmitted from the analysis device 50A in association with the user data D2. The medical information data storage unit 74c is a storage area for storing the medical information data D6. The medical information data storage unit 74c stores the medical information data D6 including the determination result related to the cognitive function of the user, the treatment program, and the information related to the consultation recommendation for the disease related to the cognitive function according to the operation of the operation input device constituting the HMI unit 61.

The data processing unit 75b receives the determination result data D5 associated with the user data D2 from the analysis device 50A via the communication unit 72, stores the user data D2 in the user data storage unit 74a while maintaining a state in which the user data D2 and the determination result data D5 are associated with each other, and stores the determination result data D5 in the determination result data storage unit 74b. By holding the association between the user data D2 and the determination result data D5, the user corresponding to the determination result data D5 can be specified in the medical institution.

An outline of the overall configuration of the abnormality determination system 1 according to the present embodiment has been described above.

### <Flow of Data in Abnormality Determination System>

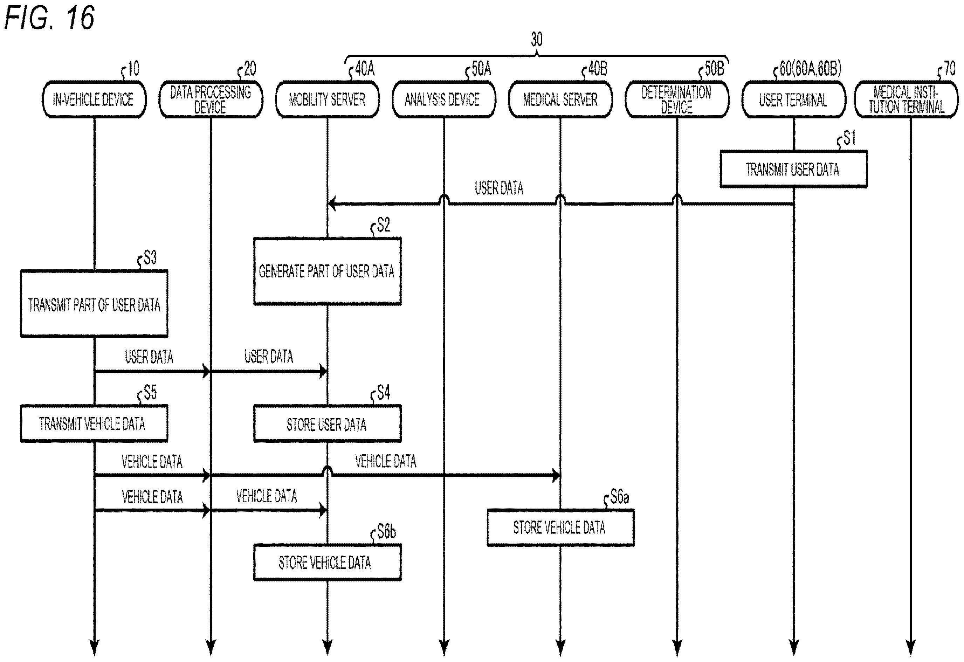

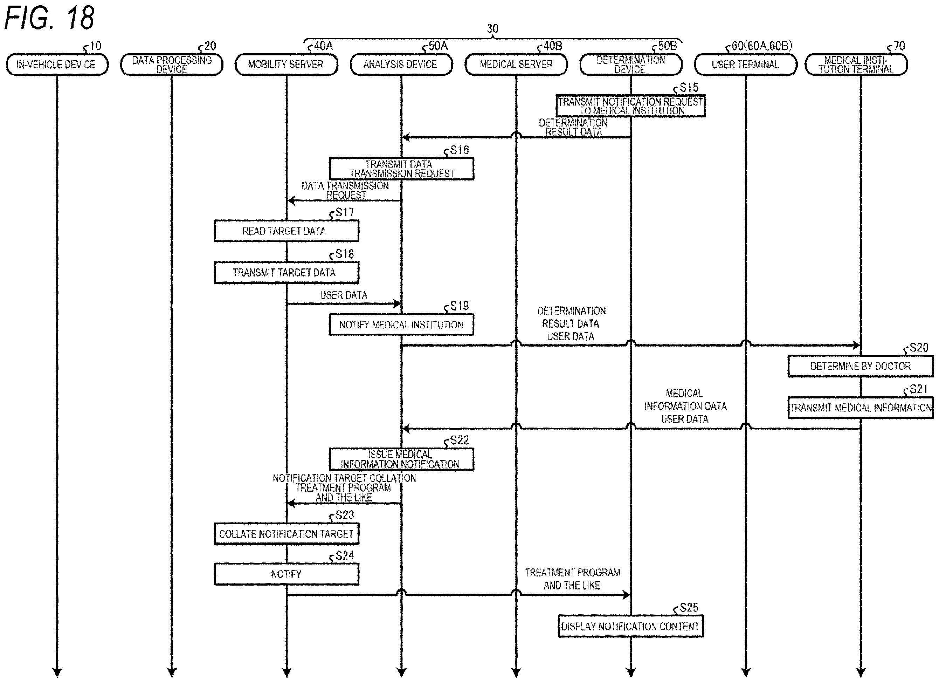

Next, an example of a flow of each process in the abnormality determination system 1 according to the present embodiment will be described with reference to FIGS. 16 to 18. In the following description, transmission and reception of data are performed via the network NW, but the description thereof may be omitted. In the following description, in order to mainly focus on the flow of data, a description of specific processing operations related to the processing unit and the like in each device may be omitted as appropriate, and the outline of the entire configuration described above will be referred to as necessary. The flow of each process described below is merely an example of a representative process, and the present invention is not limited thereto.

### <Storage of User Data and Vehicle Data>

First, as shown in FIG. 16, the user terminal 60 (first user terminal 60A) transmits, as the user data D2 to the mobility server 40A, data related to the user basic information D20, the service setting information D23, the favorite information D25, and the like registered in the user terminal 60 (first user terminal 60A) in the user data D2 (step S1), for example. The user terminal 60 (first user terminal 60A) is capable of transmitting not only the initially registered user data D2 but also the user data D2 updated after the initial registration to the mobility server 40A. The user terminal 60 (first user terminal 60A) can transmit, for example, the user basic information D20 including the terminal registration information of the second user terminal 60B as the user data D2 such that the medical information notification for the user is also notified to the second user terminal 60B used by the family of the user, the related person, or the like. In a case where the user newly registers the service related to the notification of the consultation recommendation, in a case where the registration information is changed for the service related to the notification of consultation recommendation which has already been registered, or the like, it is possible to register the vehicle identification information D10 in the user data D2 by including the vehicle identification information D10 in the terminal registration information using the user terminal 60 (first user terminal 60A). Accordingly, even when the vehicle data D1 is not stored in the mobility server 40A, the user data D2 and the determination result data D5 of the medical server 40B can be managed in association with each other.

In response to receiving the user data D2 from the user terminal 60 (first user terminal 60A), the mobility server 40A automatically generates a part of the user data D2 based on the received user data D2 (step S2). Here, the mobility server 40A automatically generates data related to the user attribute information D21, the agreement history information D22, the contract package information D24, and the like based on data related to the user basic information D20, the service setting information D23, the favorite information D25, and the like received from the user terminal 60, for example.

The user data D2 may be registered, for example, via the in-vehicle device 10. In this case, the in-vehicle device 10 is capable of registering a part of the information of the user data D2 in accordance with the operation on the HMI unit 12 performed by the user, transmits data related to the registered part of the information as the user data D2 to the data processing device 20, and transmits the data to the mobility server 40A via the data processing device 20 (step S3).

The mobility server 40A stores the received user data D2 and the automatically generated user data D2 in the server storage unit 43 (user data storage unit 43c) in association with the user ID D70 (step S4).

The in-vehicle device 10 transmits the vehicle data D1 detected and acquired by the detection and acquisition unit 11 to the data processing device 20 at an appropriate timing (step S5). In this case, the vehicle data D1 includes the vehicle identification information D10. In response to receiving the vehicle data D1 from the in-vehicle device 10, the data processing device 20 transmits the received vehicle data D1 to the mobility server 40A and the medical server 40B.

Here, the vehicle data D1 transmitted to the medical server 40B may include at least the traveling information D13 of a type used in the determination device 50B to detect the abnormal driving of the user, and information (for example, vehicle identification information D10) used when specifying the determination result data D5 generated in detecting the abnormal driving of the user.

The medical server 40B stores the vehicle data D1 received from the data processing device 20 in the server storage unit 43 (vehicle data storage unit 43a) (step S6a). In response to receiving the vehicle data D1 from the data processing device 20, the mobility server 40A stores the vehicle data D1 in the server storage unit 43 (vehicle data storage unit 43a) in association with the user ID D70 and the user data D2 (step S6b). In this case, since the user ID D70 and the vehicle ID D71 are associated with each other, the vehicle data D1 can be stored in the server storage unit 43 (user data storage unit 43c) in association with the user data D2 based on the vehicle identification information D10.

### <Determination of Abnormal Indication of User and Medical Information Notification>

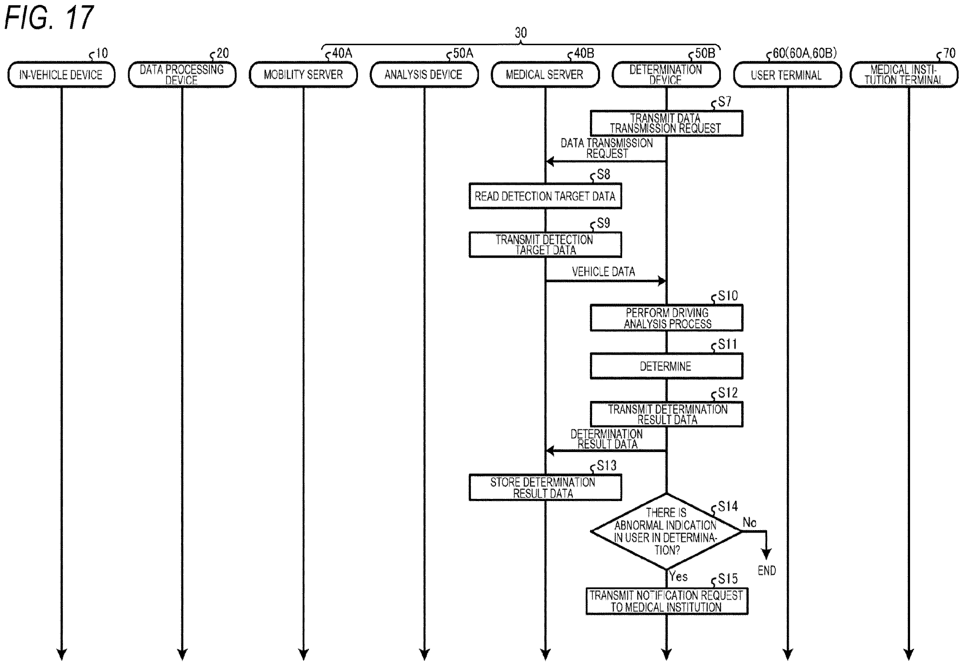

As shown in FIG. 17, in response to acquiring the vehicle data D1 used to determine an abnormal indication of the user from the medical server 40B, the determination device 50B transmits a data transmission request to the medical server 40B (step S7). In response to receiving the data transmission request from the determination device 50B, the medical server 40B reads the target vehicle data D1 (step S8). Then, the medical server 40B transmits the read vehicle data D1 to the determination device 50B (step S9). In the present embodiment, the medical server 40B transmits the vehicle data D1 including at least the vehicle identification information D10 and the traveling information D13 to the determination device 50B. In response to receiving the vehicle data D1, the determination device 50B analyzes the driving situation of the user from the vehicle data D1 and generates the driving analysis data D4 including an analysis result (step S10). Then, the determination device 50B determines the presence or absence, the content, and the like of the abnormal indication of the user based on the driving analysis data D4 (step S11). The determination device 50B generates the determination result data D5 including the information related to the determination result, and stores the determination result data D5 in the determination storage unit 54B. Here, the determination device 50B stores, in the determination storage unit 54B, the determination result data D5 in association with the vehicle identification information D10 included in the vehicle data D1. Thereafter, the determination device 50B transmits the generated determination result data D5 to the medical server 40B (step S12). Here, when the determination device 50B transmits the determination result data D5 to the medical server 40B, the medical server 40B collates the determination result data D5 received from the determination device 50B with the vehicle data D1 and the like stored in the medical server 40B, and transmits the data in a manner of being able to be stored in association with each other. Specifically, for example, the determination device 50B transmits the determination result data D5 in association with the vehicle identification information D10 included in the vehicle data D1 to the medical server 40B. Then, the medical server 40B collates the determination result data D5 received from the determination device 50B with the vehicle identification information D10 included in the vehicle data D1 stored in the medical server 40B, and stores and manages the above various kinds of data in association with each other in the server storage unit 43 (step S13). Similarly to the determination result data D5, the determination device 50B is capable of transmitting, to the medical server 40B, the driving analysis data D4 in a manner of being collated with the vehicle data D1 and the like stored in the medical server 40B and being able to be stored in association with each other.

The determination device 50B confirms the presence or absence, the content, and the like of the abnormal indication of the user based on the determination result data D5 (step S14). Here, if the determination device 50B confirms that there is no abnormal indication in the user based on the determination result data D5, the process is ended until a next data transmission request is transmitted. If the determination device 50B confirms that there is an abnormal indication in the user based on the determination result data D5, the determination device 50B transmits, to the analysis device 50A, a notification request to the medical institution together with the determination result data D5 (step S15). The notification request to the medical institution is a request to the analysis device 50A to transmit the determination result data D5 and the user data D2 corresponding to the determination result data D5 in association with each other to the medical institution terminal of the medical institution. The determination device 50B transmits the determination result data D5 and the vehicle identification information D10 corresponding to the determination result data D5 in association with each other to the analysis device 50A. In response to receiving the notification request to the medical institution from the determination device 50B, the analysis device 50A requests the mobility server 40A to transmit the user data D2 corresponding to the determination result data D5 received from the determination device 50B to the analysis device 50A (step S16).

The mobility server 40A utilizes the vehicle identification information D10 associated with the determination result data D5 received by the analysis device 50A to read the user data D2 corresponding to the determination result data D5 (step S17). Then, the mobility server 40A transmits the read user data D2 to the analysis device 50A (step S18). In this case, the mobility server 40A transmits the read user data D2 in association with the vehicle identification information D10 corresponding to the user data D2 to the analysis device 50A. The analysis device 50A associates the determination result data D5 with the user data D2 based on the vehicle identification information D10. The analysis device 50A transmits the determination result data D5 and the user data D2 corresponding to the determination result data D5 in association with each other to the medical institution terminal 70 (step S19).

In this case, the analysis device 50A may perform, with respect to the user, an agreement confirmation for transmitting the user data D2 and the determination result data D5 to the medical institution terminal 70 of the medical institution. When performing the agreement confirmation, the analysis device 50A specifies the user terminal 60 (first user terminal 60A) of the user performing the agreement confirmation based on the user data D2, and notifies the user terminal 60 (first user terminal 60A) of the agreement confirmation. In response to an operation from the user, the user terminal 60 (first user terminal 60A) transmits, to the analysis device 50A, a response indicating whether to agree to transmit the user data D2 and the determination result data D5 to the medical institution terminal 70 of the medical institution. The analysis device 50A transmits the user data D2 and the determination result data D5 to the medical institution terminal 70 of the medical institution only when the user agrees to transmit the user data D2 and the determination result data D5 to the medical institution terminal 70 of the medical institution.

In response to receiving the user data D2 and the determination result data D5 from the analysis device 50A, the medical institution terminal 70 stores the user data D2 and the determination result data D5 in association with each other in the medical institution storage unit 74 and manages the data. The medical institution terminal 70 processes the user data D2 and the determination result data D5 to be utilizable by the medical worker such as a doctor in the medical institution. The processed user data D2 and the determination result data D5 are utilized by the medical worker in the medical institution to determine the presence or absence, the content, and the like of the indication or the symptom of the disease related to the cognitive function of the user. The medical worker such as a doctor in the medical institution determines the presence or absence, the content, and the like of the indication or the symptom of the disease related to the cognitive function of the user based on the determination result data D5, the opinion of the medical worker himself or herself, and the like (step S20). Then, the medical worker such as a doctor in the medical institution creates the medical information data D6 based on the determination result, and stores the medical information data D6 in the medical institution storage unit 74 of the medical institution terminal 70. Here, the medical institution terminal 70 stores the medical information data D6 in association with the user data D2 and the determination result data D5 in the medical institution storage unit 74. Then, the medical institution terminal 70 transmits the medical information data D6 to the analysis device 50A (step S21). Here, when the medical institution terminal 70 transmits the medical information data D6 to the analysis device 50A, the analysis device 50A collates the medical information data D6 received from the medical institution terminal 70 with the user data D2 or the like stored in the analysis device 50A, and transmits the data in a manner of being able to be stored in association with each other. Specifically, for example, the medical institution terminal 70 transmits the medical information data D6 to the analysis device 50A in association with the user data D2 corresponding to the medical information data D6. Then, the analysis device 50A stores, in the analysis storage unit 54A, the medical information data D6 received from the medical institution terminal 70 in association with the user data D2 and the like corresponding to the medical information data D6, and manages the data. In this case, the analysis device 50A may specify the vehicle identification information D10 from the user data D2 corresponding to the medical information data D6 received from the medical institution terminal 70, transmit the vehicle identification information D10 and the medical information data D6 in association with each other to the mobility server 40A, and store the data in the server storage unit 43 of the mobility server 40A.

In response to receiving the medical information data D6 from the medical institution terminal 70, the analysis device 50Anotifies the user terminal 60 of the medical information including the content based on the medical information data D6. Specifically, the analysis device 50A transmits the medical information including the content based on the medical information data D6 to the mobility server 40A, and issues the medical information notification (step S22). In response to receiving the medical information from the analysis device 50A, the mobility server 40A performs collation on a target to be notified of the medical information (step S23). By collating the notification target, the user terminal 60 of the user to be notified of the medical information is specified. Here, when issuing the medical information notification to the user terminal 60 via the mobility server 40A, the analysis device 50A transmits, to the mobility server 40A, the medical information including the treatment program and the like based on the medical information data D6 in a state in which the mobility server 40A can specify the user terminal 60 to which the medical information notification is issued. For example, the analysis device 50A transmits, to the mobility server 40A, the medical information including the treatment program and the like based on the medical information data D6 and the user data D2 corresponding to the medical information data D6 in association with each other, so that the mobility server 40A can specify the user terminal 60 of the user to be notified of the medical information based on the user data D2 received from the analysis device 50A. For example, the analysis device 50A transmits, to the mobility server 40A, the medical information including the treatment program and the like based on the medical information data D6 and the vehicle identification information D10 included in the vehicle data D1 corresponding to the medical information data D6 in association with each other, so that the mobility server 40A may collate the user data D2 stored in the mobility server 40Abased on the vehicle identification information D10 received from the analysis device 50A, and specify the user terminal 60 of the user to be notified of the medical information from the user data D2.

When specifying the user terminal 60 to be notified of the medical information, the mobility server 40A transmits the medical information notification to the specified user terminal 60 (step S24). In response to receiving the medical information notification from the mobility server 40A, the user terminal 60 outputs the content corresponding to the medical information notification to the display unit (step S25). For example, when the content of the medical information notification is a determination result related to the cognitive function of the user, information related to the presence or absence, the content, and the like of the indication or the symptom of the disease related to the cognitive function of the user is output to the user terminal 60. When the content of the medical information notification includes the treatment program, information related to the treatment program determined to be suitable for the user by the medical worker of the medical institution is output to the user terminal 60. When the content of the medical information notification includes the consultation recommendation on the disease related to the cognitive function, the information related to the consultation recommendation as shown in FIGS. 12 to 14 is output.

### <Effects of First Embodiment>

As described above, the abnormality determination system 1 according to the present embodiment includes: the mobility server 40A that stores the user data D2 related to the user of the vehicle V; the medical server 40B that stores the vehicle data D1 including the traveling information D13 of the vehicle V; the determination and analysis devices 50A, 50B that determine the abnormal indication of the user from the vehicle data D1, store the determination result data D5 including a determination result in the medical server 40B, and issue the notification of the medical information to the user based on the determination result data D5; and the user terminals 60, 60A, 60B that are capable of receiving the notification from the determination and analysis devices 50A, 50B. The mobility server 40A and the medical server 40B cooperate with each other to manage the user data D2, the vehicle data D1, and the determination result data D5 in association with each other, and the determination and analysis devices 50A, 50B transmit at least the determination result data D5 to the medical institution terminal 70 of the medical institution, receive the medical information data D6 created based on the determination result data D5 and the opinion of the medical worker at the medical institution from the medical institution terminal 70, and notify the user terminals 60, 60A, 60B of the medical information including the content based on the medical information data D6.

The abnormality determination system 1 according to the present invention performs determination related to the abnormal indication of the user from the vehicle data D1 collected by the in-vehicle device 10, and provides the determination result data including the determination result to the medical institution, and thus, the presence or absence of the indication, the symptom, or the like of deterioration in cognitive function can be determined in the medical institution from the driving situation of the vehicle V which is a daily behavior of the user. Accordingly, it is possible to discover the indication, the symptom, or the like of deterioration in cognitive function of the user at an early stage. In the abnormality determination system 1 according to the present embodiment, the medical worker such as a doctor in the medical institution can determine a disease and the like related to the cognitive function of the user based on the determination result data D5 including the information related to the abnormal driving of the user, and thus, unlike a simple inspection related to the cognitive function in the related art, rather than making a determination based on a temporary state of a person taking such a simple inspection, it is possible to determine the presence or absence, the content, and the like of the disease related to the cognitive function of the user based on the driving situation of the vehicle V which is a daily behavior of the user. Accordingly, as compared with the simple inspection as described above, since it is possible to determine the indication or the symptom of deterioration in cognitive function at an appropriate time, in particular, it is possible to detect an abnormality in the cognitive function at an initial stage. The abnormality determination system 1 according to the present embodiment is configured to manage the user data D2 by the mobility server 40A, and manage the determination result data D5, which is information having high confidentiality and related to the body of the user, by the medical server 40B different from the mobility server 40A. According to this configuration, the user data D2 including the personal information and the determination result data D5 having high confidentiality are managed separately so as not to be directly associated with each other, and thus, a secret retention can be improved and various kinds of data can be smoothly utilized. Since the mobility server 40A and the medical server 40B are physically separated, each server can acquire various kinds of data at a suitable timing according to the use. According to this configuration, it is possible to effectively use a storage resource. In addition, in the abnormality determination system 1 according to the present embodiment, since the server is divided according to the use, it is possible to acquire various kinds of data in the data type or the suitable timing according to the use. Accordingly, the server can be maintained without affecting each other, and the storage resource can be effectively used.

In the abnormality determination system 1 according to the present embodiment, the mobility server 40A is disposed in the mobility base station 101 that relays communication between the vehicle V and the user terminals 60, 60A, 60B, and the medical server 40B is disposed in the medical base station 102 that relays communication between the vehicle V and the medical institution terminal 70 and is a base station different from the mobility base station 101.

In the abnormality determination system 1 according to the present embodiment, the mobility server 40A and the medical server 40B are provided in different base stations (mobility base station 101, medical base station 102). According to this configuration, it is possible to manage the data stored in the server according to the degree of confidentiality. For example, the medical institution treats the information related to the body, such as a medical record of a patient, and thus, higher information security is required in data management. The medical institution that requires high information security manages the medical base station 102, and the medical server 40B is disposed in the medical base station 102, so that confidentiality of the determination result data D5 can be improved. In this way, by classifying the server and the base station according to the type of data, appropriate information management can be performed.

In the abnormality determination system 1 according to the present embodiment, the notification unit receives the medical information data D6 created based on the determination result data D5 and the opinion of the medical worker in the medical institution from the medical institution terminal 70, specifies the user terminals 60, 60A, 60B from the user data D2 corresponding to the medical information data D6, and issues the medical information notification, which is a notification of the content based on the medical information data D6, to the user terminals 60, 60A, 60B.

The abnormality determination system 1 according to the present embodiment notifies the user (user terminal 60) of the medical information created based on the determination result related to the abnormal indication of the user and the opinion of the medical worker of the medical institution, so that treatment for the disease related to the cognitive function for the user can be started early.

In the abnormality determination system 1 according to the present embodiment, the medical information data D6 includes information related to at least one of a treatment program determined to be suitable for the user by the medical worker, the determination result related to the cognitive function of the user, and consultation recommendation for the disease related to the cognitive function.

The abnormality determination system 1 according to the present embodiment notifies the user (user terminal 60) of the treatment program determined to be suitable for the user by the medical worker such as a doctor of the medical institution, thereby allowing the user to know a specific treatment plan or the like and, for example, to improve an intention of the user to consult the medical institution. By notifying the user (user terminal 60) of the determination result related to the cognitive function of the user, the user can know the cognitive function state of himself or herself and a necessity of treatment, and the intention of the user to consult the medical institution can be improved. By issuing the user of the notification of the consultation recommendation on the disease related to the cognitive function, it is possible to improve the intention of the user to consult the medical institution.

In the abnormality determination system 1 according to the present embodiment, in a case where the medical information data D6 includes the information related to the consultation recommendation, the determination and analysis devices 50A, 50B transmit the medical information including the presentation of the behavior recommended to the user to the user terminals 60, 60A, 60B, and the user terminals 60, 60A, 60B present the behavior recommended to the user and display the operation of executing the behavior in a receivable manner.

The abnormality determination system 1 according to the present embodiment presents a specific behavior recommended to the user, and is provided with an input unit (icons 60a to 60d) capable of executing the behavior, so that it is possible to improve the intention of the user to consult the medical institution. Deterioration in cognitive function may not appear in an external wound, and for example, the user may not know which department the specialist corresponds to, or may be hard to visit a comprehensive hospital for an initial inspection. Even in such a case, the user terminal 60 displays the input unit (the icons 60a to 60d) in an operable manner, so that the user can smoothly take the recommended behavior. That is, by providing the input unit (icons 60a to 60d) in the user terminal 60 in an operable manner, it is possible to reduce a psychological barrier when the user makes a reservation for the medical institution or the like.

In the abnormality determination system 1 according to the present embodiment, the traveling information D13 includes the information related to the driving operation of the vehicle V, and the determination and analysis devices 50A, 50B determine the abnormal indication of the user based on the determination reference data D8 including the correlation relation between the driving operation of the vehicle V and the cognitive function state of the user

In the abnormality determination system 1 according to the present embodiment, the determination device 50B primarily determines the presence or absence, the content, and the like of the abnormal indication of the user based on the vehicle data D1 collected by the in-vehicle device 10, and if it is determined that there is an abnormal indication in the user, the determination device 50B provides the information related to the determination result to the medical institution. Accordingly, it is possible to narrow down information to be processed by the medical worker of the medical institution to important information. The determination related to the presence or absence, the content, and the like of the abnormal indication of the user is not performed only by the determination device 50B, but the medical worker of the medical institution performs a secondary determination with respect to the result of the primary determination performed by the determination device 50B in consideration of a past medical history and the like of the user, so that the determination with higher accuracy can be performed. In this way, it is possible to discover the indication or the symptom of the deterioration in cognitive function from the driving situation of the vehicle V which is the daily behavior of the user at an early stage.

In the abnormality determination system 1 according to the present embodiment, the mobility server 40A and the medical server 40B manage the user data D2, the vehicle data D1, and the determination result data D5 in association with each other for each user, and the user data D2 includes information that can be collated with diagnosis information of a patient stored at the medical institution.

The abnormality determination system 1 according to the present embodiment includes the information that can be collated with the diagnosis information of the patient stored in the medical institution in the user data D2, thereby enabling the medical worker of the medical institution to determine the indication, the symptom, or the like of the disease related to the cognitive function while referring to the past and current diagnosis information of the user. Accordingly, the medical information having more detailed contents can be notified to the user (user terminal 60).

In the abnormality determination system 1 according to the present embodiment, the storage unit is configured to receive access from the medical institution terminal 70.

In the abnormality determination system 1 according to the present embodiment, since the medical server 40B that constitutes the storage unit is configured to receive access from the medical institution terminal 70, the medical worker such as a doctor of the medical institution can utilize data (for example, test result data DX) stored in the storage unit at the time of, for example, diagnosis of the user.

In the abnormality determination system 1 according to the present embodiment, the vehicle data D1 includes the vehicle identification information D10 that identifies vehicle V, the mobility server 40A further manages the vehicle data D1 and stores the user data D2 and the vehicle identification information D10 in association with each other, the medical server 40B stores the determination result data D5 and the vehicle identification information D10 in association with each other, and the determination and analysis devices 50A, 50B transmit, to the medical institution terminal 70, the user data D2 and the determination result data D5 in association with each other based on the vehicle identification information D10.

By associating the user data D2 with the determination result data D5 via the vehicle identification information D10, the abnormality determination system 1 according to the present embodiment can manage the user data D2 managed by the mobility server 40A and the determination result data D5 and the like managed by the medical server 40B on different servers without directly correlating and managing the data, and thus, confidentiality of data management can be improved. The abnormality determination system 1 according to the present embodiment can be used for, in addition to the notification of the medical information issued by the service providing device 30, various services and analyses by enabling the vehicle data D1 managed by the mobility server 40A and the analysis device 50A to be utilized by an agency (dealer) or a developer of the vehicle V For example, the mobility server 40A and the analysis device 50A are used for a service related to maintenance of the vehicle V Since the mobility server 40A and the medical server 40B are physically separated, each server can acquire the vehicle data D1 at a suitable circle according to the use. According to this configuration, it is possible to effectively use a storage resource. In addition, in the abnormality determination system 1 according to the present embodiment, since the server is divided according to the use, it is possible to acquire the vehicle data D1 in the data type or the suitable cycle according to the use. Accordingly, the server can be maintained without affecting each other, and the storage resource can be effectively used.

In addition, in the abnormality determination system 1 according to the present embodiment, the medical server 40B directly acquires the vehicle data D1 from the outside without synchronizing with the mobility server 40A.

In the abnormality determination system 1 according to the present embodiment, the medical server 40B directly acquires the vehicle data D1 from outside without synchronizing with the mobility server 40A, so that an information type, a reception circle, a data amount, and the like included in the vehicle data D1 can be configured in a format suitable for each server, without depending on the server, for example.

The base station device (service providing device 30) according to the present embodiment includes: the mobility server 40A that stores the user data D2 related to the user of the vehicle V; the medical server 40B that stores the vehicle data D1 including the traveling information D13 of the vehicle V; and the determination and analysis devices 50A, 50B that determine the abnormal indication of the user from the vehicle data D1, store the determination result data D5 including a determination result in the medical server 40B, and issue the notification of the medical information to the user based on the determination result data D5. The mobility server 40A and the medical server 40B cooperate with each other to manage the user data D2, the vehicle data D1, and the determination result data D5 in association with each other, and the determination and analysis devices 50A, 50B transmit at least the determination result data D5 to the medical institution terminal 70 of the medical institution, receive the medical information data D6 created based on the determination result data D5 and the opinion of the medical worker at the medical institution from the medical institution terminal 70, and notify the user terminals 60, 60A, 60B of the medical information including the content based on the medical information data D6.

The base station device (service providing device 30) according to the present invention performs determination related to the abnormal indication of the user from the vehicle data D1 collected by the in-vehicle device 10, and provides the determination result data including the determination result to the medical institution, and thus, the presence or absence of the indication, the symptom, or the like of deterioration in cognitive function can be determined in the medical institution from the driving situation of the vehicle V which is a daily behavior of the user. Accordingly, it is possible to discover the indication, the symptom, or the like of deterioration in cognitive function of the user at an early stage. In the base station device (service providing device 30) according to the present embodiment, the medical worker such as a doctor in the medical institution can determine the disease and the like related to the cognitive function of the user based on the determination result data D5 including the information related to the presence or absence, the content, and the like of the abnormal indication of the user, and thus, unlike a simple inspection related to the cognitive function in the related art, rather than making a determination based on a temporary state of a person taking such a simple inspection, it is possible to determine the presence or absence, the content, and the like of the disease related to the cognitive function of the user based on the driving situation of the vehicle V which is a daily behavior of the user. Accordingly, as compared with the simple inspection as described above, since it is possible to determine the indication or the symptom of deterioration in cognitive function at an appropriate time, in particular, it is possible to detect an abnormality in the cognitive function at an initial stage. The base station device (service providing device 30) according to the present embodiment is configured to manage the user data D2 by the mobility server 40A, and manage the determination result data D5, which is the information having high confidentiality and related to the body of the user, by the medical server 40B different from the mobility server 40A. According to this configuration, the user data D2 including the personal information and the determination result data D5 having high confidentiality are managed separately so as not to be directly associated with each other, and thus, a secret retention can be improved and various kinds of data can be smoothly utilized. Since the mobility server 40A and the medical server 40B are physically separated, each server can acquire various kinds of data at a suitable timing according to the use. According to this configuration, it is possible to effectively use a storage resource. In addition, in the base station device (service providing device 30) according to the present embodiment, since the server is divided according to the use, it is possible to acquire various kinds of data in the data type or the suitable timing according to the use. Accordingly, the server can be maintained without affecting each other, and the storage resource can be effectively used.

### <Second Embodiment>

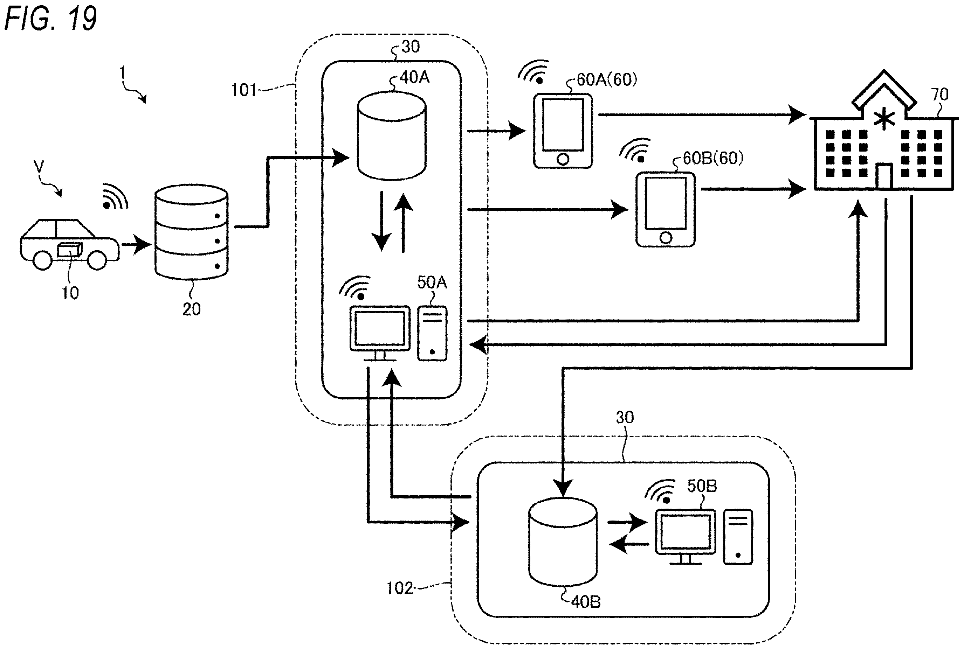

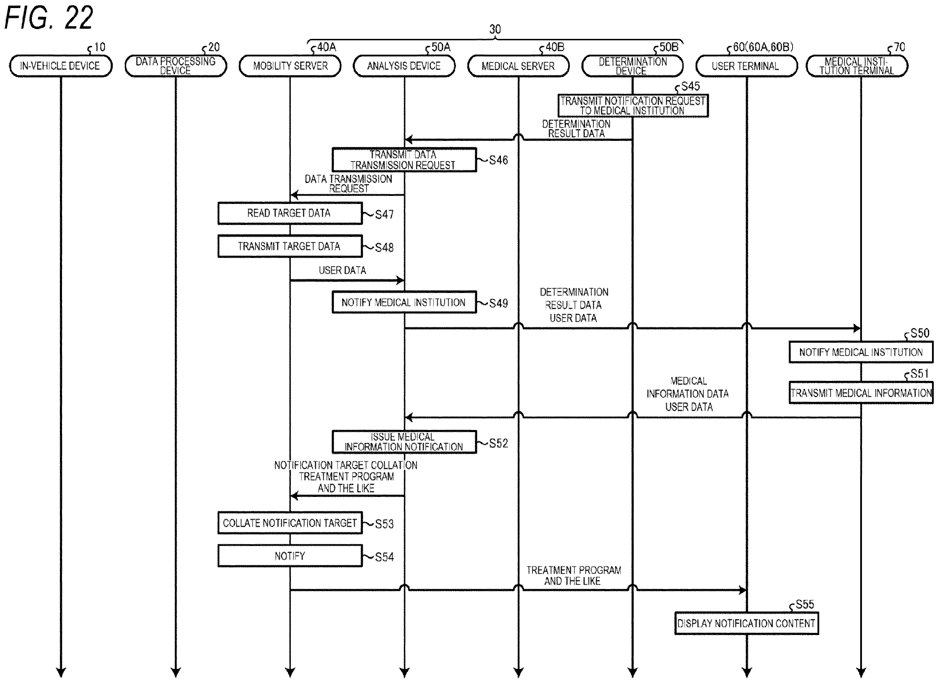

Next, a second embodiment will be described with reference to FIGS. 19 to 22. As shown in FIG. 19, the abnormality determination system 1 and a base station device (service providing device 30) according to the second embodiment are different from the abnormality determination system 1 and the base station device (service providing device 30) according to the first embodiment described above in that the vehicle data D1 is transmitted from the data processing device 20 to the medical server 40B via the mobility server 40A and the analysis device 50A. The analysis device 50A receives the vehicle data D1 transmitted from the mobility server 40A, and stores the vehicle data D1 in the vehicle data storage unit 54a. The analysis device 50A transmits the stored vehicle data D1 to the medical server 40B. Other configurations are substantially the same as those of the first embodiment.

### <Flow of Data in Abnormality Determination System>

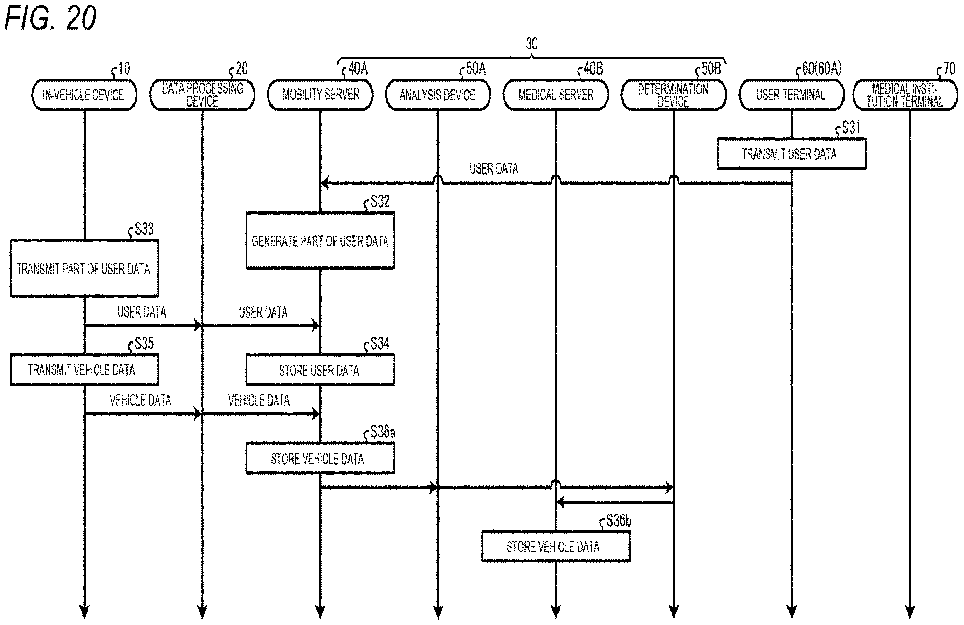

Next, an example of a flow of each process in the abnormality determination system 1 according to the second embodiment will be described with reference to FIGS. 20 to 22. Similarly to the first embodiment, in the following description, transmission and reception of data are performed via the network NW, but the description thereof may be omitted. In the following description, in order to mainly focus on the flow of data, a description of specific processing operations related to the processing unit and the like in each device may be omitted as appropriate, and the outline of the entire configuration described above will be referred to as necessary. The flow of each process described below is merely an example of a representative process, and the present invention is not limited thereto.

### <Storage of User Data and Vehicle Data>

First, as shown in FIG. 20, the user terminal 60 (first user terminal 60A) transmits, as the user data D2 to the mobility server 40A, data related to the user basic information D20, the service setting information D23, the favorite information D25, and the like registered in the user terminal 60 (first user terminal 60A) in the user data D2 (step S31), for example. The user terminal 60 (first user terminal 60A) is capable of transmitting not only the initially registered user data D2 but also the user data D2 updated after the initial registration to the mobility server 40A. The user terminal 60 (first user terminal 60A) can transmit, for example, the user basic information D20 including the terminal registration information of the second user terminal 60B as the user data D2 such that the notification of the consultation recommendation for the user is also notified to the second user terminal 60B mainly used by the family of the user, the related person, or the like. In a case where the user newly registers a service related to the notification of the consultation recommendation, in a case where the registration information is changed for the service related to the notification of the consultation recommendation that has already been registered, or the like, it is possible to register the vehicle identification information D10 in the user data D2 by including the vehicle identification information D10 in the terminal registration information using the user terminal 60 (first user terminal 60A). Accordingly, even when the vehicle data D1 is not stored in the mobility server 40A, the user data D2 and the determination result data D5 can be managed in association with each other based on the vehicle identification information D10.

In response to receiving the user data D2 from the user terminal 60 (first user terminal 60A), the mobility server 40A automatically generates a part of the user data D2 based on the received user data D2 (step S32). Here, the mobility server 40A automatically generates data related to the user attribute information D21, the agreement history information D22, the contract package information D24, and the like based on data related to the user basic information D20, the service setting information D23, the favorite information D25, and the like received from the user terminal 60 (first user terminal 60A), for example.

The user data D2 may be registered, for example, via the in-vehicle device 10. In this case, the in-vehicle device 10 can register a part of the information of the user data D2 in accordance with the operation on the HMI unit 12 performed by the user, and also can transmit data related to the registered part of the information as the user data D2 to the data processing device 20, and transmit the data to the mobility server 40A via the data processing device 20 (step S33).

The mobility server 40A stores the received user data D2 and the automatically generated user data D2 in the server storage unit 43 (user data storage unit 43c) in association with the corresponding user ID D70 based on the ID data D7 and the like stored in the server storage unit 43 (ID data storage unit 43b) (step S34).

The in-vehicle device 10 transmits the vehicle data D1 detected and acquired by the detection and acquisition unit 11 to the data processing device 20 at an appropriate timing (step S35). In this case, the in-vehicle device 10 transmits the vehicle data D1 to the data processing device 20. In this case, the vehicle data D1 includes the vehicle identification information D10, the DCM information D11, the vehicle basic information D12, the traveling information D13, and the like. In response to receiving the vehicle data D1 from the in-vehicle device 10, the data processing device 20 transmits the received vehicle data D1 to the mobility server 40A.

In response to receiving the vehicle data D1 from the data processing device 20, the mobility server 40A stores, in the server storage unit 43 (vehicle data storage unit 43a), the vehicle data D1 and the user ID D70 and the user data D2 corresponding to the vehicle data D1 in association with each other based on the ID data D7 and the like stored in the server storage unit 43 (ID data storage unit 43b) and the vehicle identification information D10 (step S36a). The mobility server 40A transmits the vehicle data D1 to the medical server 40B via the analysis device 50A and the determination device 50B. Here, the vehicle data D1 transmitted from the mobility server 40A to the medical server 40B includes at least the traveling information D13 used in the determination device 50B to perform the determination related to the abnormal indication of the user, and the vehicle identification information D10 used when specifying the determination result data D5 generated in performing the determination related to the abnormal indication of the user. In response to receiving the vehicle data D1, the medical server 40B stores the vehicle data D1 in the server storage unit 43 (vehicle data storage unit 43a) (step S36b).

### <Determination of Abnormal Indication of User and Medical Information Notification>

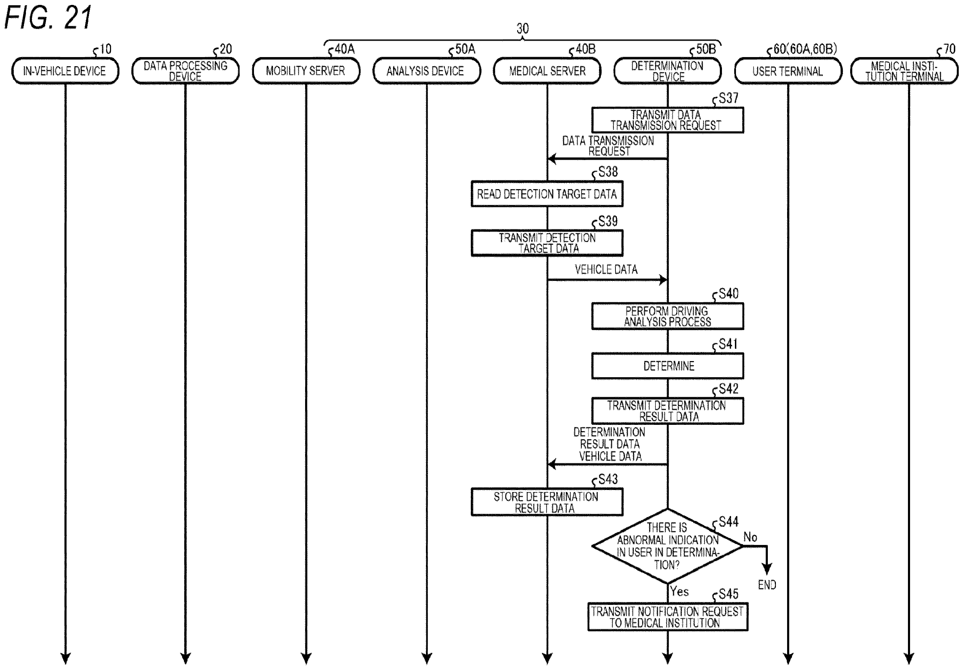

As shown in FIG. 21, in response to acquiring the vehicle data D1 used to determine an abnormal indication of the user from the medical server 40B, the determination device 50B transmits a data transmission request to the medical server 40B (step S37). In response to receiving the data transmission request from the determination device 50B, the medical server 40B reads the target vehicle data D1 (step S38). Then, the medical server 40B transmits the vehicle data D1 including at least the vehicle identification information D10 and the traveling information D13 to the determination device 50B (step S39). In response to receiving the vehicle data D1, the determination device 50B analyzes the driving situation of the user from the vehicle data D1 and generates the driving analysis data D4 including an analysis result (step S40). Then, the determination device 50B determines the presence or absence, the content, and the like of the abnormal indication of the user in the vehicle V based on the driving analysis data D4 (step S41). The determination device 50B generates the determination result data D5 including the information related to the determination result, and stores the determination result data D5 in the determination storage unit 54B. Here, the determination device 50B stores, in the determination storage unit 54B, the determination result data D5 in association with the vehicle identification information D10 included in the vehicle data D1. Thereafter, the determination device 50B transmits the generated determination result data D5 to the medical server 40B (step S42). Here, when the determination device 50B transmits the determination result data D5 to the medical server 40B, the medical server 40B collates the determination result data D5 received from the determination device 50B with the vehicle data D1 and the like stored in the medical server 40B, and transmits the data in a manner of being able to be stored in association with each other. Specifically, for example, the determination device 50B transmits the determination result data D5 in association with the vehicle identification information D10 included in the vehicle data D1 to the medical server 40B. Then, the medical server 40B collates the determination result data D5 received from the determination device 50B with the vehicle identification information D10 included in the vehicle data D1 stored in the medical server 40B, and stores and manages the above various kinds of data in association with each other in the server storage unit 43 (step S43). Similarly to the determination result data D5, the determination device 50B is capable of transmitting, to the medical server 40B, the driving analysis data D4 in a manner of being collated with the vehicle data D1 and the like stored in the medical server 40B and being able to be stored in association with each other.

Then, the determination device 50B confirms the presence or absence, the content, and the like of the abnormal indication of the user based on the determination result data D5 (step S44). Here, if the determination device 50B confirms that there is no abnormal indication in the user of the vehicle V based on the determination result data D5, the process is ended until a next data transmission request is transmitted. If the determination device 50B confirms that there is an abnormal indication in the user based on the determination result data D5, the determination device 50B transmits, to the analysis device 50A, a notification request to the medical institution together with the determination result data D5 (step S45). The notification request to the medical institution is a request to the analysis device 50A to transmit the determination result data D5 and the user data D2 corresponding to the determination result data D5 in association with each other to the medical institution terminal of the medical institution. Here, the determination device 50B transmits the determination result data D5 and the vehicle identification information D10 corresponding to the determination result data D5 in association with each other to the analysis device 50A. In response to receiving the notification request to the medical institution from the determination device 50B, the analysis device 50A requests the mobility server 40A to transmit the user data D2 corresponding to the determination result data D5 received from the determination device 50B to the analysis device 50A (step S46).

The mobility server 40A utilizes the vehicle identification information D10 associated with the determination result data D5 received by the analysis device 50A to read the user data D2 corresponding to the determination result data D5 (step S47). Then, the mobility server 40A transmits the read user data D2 to the analysis device 50A (step S48). In this case, the mobility server 40A transmits the read user data D2 in association with the vehicle identification information D10 corresponding to the user data D2 to the analysis device 50A. The analysis device 50A associates the determination result data D5 with the user data D2 based on the vehicle identification information D10. The analysis device 50A transmits the determination result data D5 and the user data D2 corresponding to the determination result data D5 in association with each other to the medical institution terminal 70 (step S49).

In this case, similarly to the first embodiment described above, the analysis device 50A may perform, with respect to the user, the agreement confirmation for transmitting the user data D2 and the determination result data D5 to the medical institution terminal 70 of the medical institution. When performing the agreement confirmation, the analysis device 50A specifies the user terminal 60 (first user terminal 60A) of the user performing the agreement confirmation based on the user data D2, and notifies the user terminal 60 (first user terminal 60A) of the agreement confirmation. In response to an operation from the user, the user terminal 60 (first user terminal 60A) transmits, to the analysis device 50A, a response indicating whether to agree to transmit the user data D2 and the determination result data D5 to the medical institution terminal 70 of the medical institution. The analysis device 50A transmits the user data D2 and the determination result data D5 to the medical institution terminal 70 of the medical institution only when the user agrees to transmit the user data D2 and the determination result data D5 to the medical institution terminal 70 of the medical institution.

In response to receiving the user data D2 and the determination result data D5 from the analysis device 50A, the medical institution terminal 70 stores the user data D2 and the determination result data D5 in association with each other in the medical institution storage unit 74 and manages the data. The medical institution terminal 70 processes the user data D2 and the determination result data D5 to be utilizable by the medical worker such as a doctor in the medical institution. The processed user data D2 and the determination result data D5 are utilized by the medical worker in the medical institution to determine the presence or absence, the content, and the like of the indication or the symptom of the disease related to the cognitive function of the user. The medical worker such as a doctor in the medical institution determines the presence or absence, the content, and the like of the indication or the symptom of the disease related to the cognitive function of the user based on the determination result data D5, the opinion of the medical worker himself or herself, and the like (step S50). Then, the medical worker such as a doctor in the medical institution creates the medical information data D6 based on the determination result, and stores the medical information data D6 in the medical institution storage unit 74 of the medical institution terminal 70. Here, the medical institution terminal 70 stores the medical information data D6 in association with the user data D2 and the determination result data D5 in the medical institution storage unit 74. Then, the medical institution terminal 70 transmits the medical information data D6 to the analysis device 50A (step S51). Here, when the medical institution terminal 70 transmits the medical information data D6 to the analysis device 50A, the analysis device 50A collates the medical information data D6 received from the medical institution terminal 70 with the user data D2 or the like stored in the analysis device 50A, and transmits the data in a manner of being able to be stored in association with each other. Specifically, for example, the medical institution terminal 70 transmits the medical information data D6 to the analysis device 50A in association with the user data D2 corresponding to the medical information data D6. Then, the analysis device 50A stores, in the analysis storage unit 54A, the medical information data D6 received from the medical institution terminal 70 in association with the user data D2 and the like corresponding to the medical information data D6, and manages the data. In this case, the analysis device 50A may specify the vehicle identification information D10 from the user data D2 corresponding to the medical information data D6 received from the medical institution terminal 70, transmit the vehicle identification information D10 and the medical information data D6 in association with each other to the mobility server 40A, and store the data in the server storage unit 43 of the mobility server 40A.

In response to receiving the medical information data D6 from the medical institution terminal 70, the analysis device 50Anotifies the user terminal 60 of the medical information including the content based on the medical information data D6. Specifically, the analysis device 50A transmits the medical information including the content based on the medical information data D6 to the mobility server 40A, and issues the medical information notification (step S52). In response to receiving the medical information notification from the analysis device 50A, the mobility server 40A performs collation on a target to be notified of the medical information (step S53). By collating the notification target, the user terminal 60 of the user to be notified of the medical information is specified. Here, when issuing the medical information notification to the user terminal 60 via the mobility server 40A, the analysis device 50A transmits, to the mobility server 40A, the medical information including the treatment program and the like based on the medical information data D6 in a state in which the mobility server 40A can specify the user terminal 60 to which the medical information notification is issued. For example, the analysis device 50A transmits, to the mobility server 40A, the medical information including the treatment program and the like based on the medical information data D6 and the user data D2 corresponding to the medical information data D6 in association with each other, so that the mobility server 40A can specify the user terminal 60 of the user to be notified of the medical information based on the user data D2 received from the analysis device 50A. For example, the analysis device 50A transmits, to the mobility server 40A, the medical information including the treatment program and the like based on the medical information data D6 and the vehicle identification information D10 corresponding to the medical information data D6 in association with each other, so that the mobility server 40A may collate the user data D2 stored in the mobility server 40A based on the vehicle identification information D10 received from the analysis device 50A, and specify the user terminal 60 of the user to be notified of the medical information from the user data D2.

When specifying the user terminal 60 to be notified of the medical information, the mobility server 40A transmits the medical information notification to the specified user terminal 60 (step S54). In response to receiving the medical information notification from the mobility server 40A, the user terminal 60 outputs the content corresponding to the medical information notification to the display unit (step S55). For example, when the content of the medical information notification is a determination result related to the cognitive function of the user, information related to the presence or absence, the content, and the like of the indication or the symptom of the disease related to the cognitive function of the user is output to the user terminal 60. When the content of the medical information notification includes the treatment program, information related to the treatment program determined to be suitable for the user by the medical worker such as a doctor of the medical institution is output to the user terminal 60. When the content of the medical information notification includes the consultation recommendation on the disease related to the cognitive function, the information related to the consultation recommendation as shown in FIGS. 12 to 14 is output.

### <Effects of Second Embodiment>

As described above, in the abnormality determination system 1 according to the second embodiment, the medical server 40B acquires only the vehicle data D1 utilized by the determination and analysis devices 50A, 50B to perform the determination related to the abnormal indication of the user, out of the vehicle data D1 stored in the mobility server 40A.

In the abnormality determination system 1 according to the present embodiment, the medical server 40B directly acquires the vehicle data from the vehicle V via the mobility server 40A. Security can be improved by setting the specific mobility server 40A as an acquisition source of the vehicle data D1.

In the abnormality determination system 1 according to the present embodiment, the determination result data D5, which is personal information having high confidentiality, is managed by the medical server 40B, and the user data D2 is managed by the mobility server 40A different from the medical server 40B, so that it is possible to smoothly utilize data used for mobility services while improving secret retention.

The abnormality determination system 1 and the base station device according to each embodiment of the present invention are not limited to each embodiment described above, and various modifications can be made within the scope described in the claims.

### <Modification>

In the above description, the abnormality determination system 1 collates the determination result data D5 and the medical information notification corresponding to the vehicle identification information D10 between two different servers based on the vehicle identification information D10, but the present invention is not limited thereto. For example, the abnormality determination system 1 may collate the determination result data D5 or the medical information notification based on an ID of the in-vehicle device 10, a manufacturing number of the in-vehicle device 10, a registrant number of the service in the abnormality determination system 1, or the like.

In the above description, the abnormality determination system 1 is configured such that the devices constituting the system transmit and receive data to and from each other through communication, but the present invention is not limited thereto. For example, a part or all of the data treated in the abnormality determination system 1 may be written to or read from a recording medium via the data input and output units 14, 22, 42, 53, 63, 73 to transfer the data. Specifically, the vehicle data D1 collected by the in-vehicle device 10 may be written to a recording medium by the data input and output unit 14, read from the recording medium by the data input and output unit 22, and transferred to the data processing device 20. In the data processing device 20, the vehicle data D1 stored in the mobility server 40A, the medical server 40B, or the like by a worker may be written to the recording medium by the data input and output unit 22, and in the service providing device 30, the vehicle data D1 may be read from the recording medium by the data input and output unit 42 of the mobility server 40A, the medical server 40B, or the like, so that the vehicle data D1 may be collectively stored in the mobility server 40A, the medical server 40B, or the like from the data processing device 20.

In the above description, the vehicle V may be a private vehicle or the like owned or used by the user, or may be a rental car, a shared car, or the like shared by unspecified utilizers. In a case where the vehicle V is a vehicle shared by unspecified utilizers, when the utilizer reserves or registers the use of the shared vehicle V, the abnormality determination system 1 executes a process of associating the user ID D70 with the vehicle ID D71 of the shared vehicle V from the in-vehicle device 10 or the user terminal 60 to the mobility server 40A.

Each of the processing units 16, 24, 44, 55, 65, 75 described above may be implemented by a single processor, and the process related to each function may be executed, or may be implemented by a combination of a plurality of independent processors, and the process related to each function may be executed by each processor executing a program. The processes executed by the processing units 16, 24, 44, 55, 65 may be integrated into a single processing circuit or may be distributed to a plurality of processing circuits.

Each of the mobility server 40A and the analysis device 50A described above may be implemented by a single processor, and the process related to each function may be executed, or may be implemented by a combination of a plurality of independent processors, and the process related to each function may be executed by each processor executing a program.

Each of the medical server 40B and the determination device 50B described above may be implemented by a single processor, and the process related to each function may be executed, or may be implemented by a combination of a plurality of independent processors, and the process related to each function may be executed by each processor executing a program. The processes executed by the medical server 40B and the determination device 50B may be integrated into a single processing circuit or may be distributed to the plurality of processing circuits.

The abnormality determination system and the base station device according to the present embodiment may be configured by appropriately combining the components of the embodiments and the modifications described above.

In the above description, the configuration in which the mobility server 40A and the medical server 40B exchange information via the analysis device 50A has been described as an example, but the configuration is not limited thereto. For example, the mobility server 40A and the medical server 40B may exchange information without going through the analysis device 50A.

### REFERENCE SIGNS LIST

- 1: abnormality determination system
- 10: in-vehicle device
- 20: data processing device
- 30: service providing device (base station device)
- 40A: mobility server
- 40B: medical server
- 50A: analysis device (determination and analysis device)
- 50B: determination device (determination and analysis device)
- 60: user terminal
- 60A: first user terminal
- 60B: second user terminal
- 70: medical institution terminal
- NW: network
- V: vehicle

## Claims

1. An abnormality determination system (1), comprising:
a mobility server (40A) configured to store user data (D2) related to a user of a vehicle (V);
a medical server (40B) configured to store vehicle data (D1) including traveling information (D13) of the vehicle (V);
a determination and analysis device (50A, 50B) configured to determine an abnormal indication of the user from the vehicle data (D1), store determination result data (D5) including a determination result in the medical server (40B), and issue a notification of medical information to the user based on the determination result data (DS); and
a user terminal (60, 60A, 60B) configured to receive the notification from the determination and analysis device (50A, 50B), wherein
the mobility server (40A) and the medical server (40B) cooperate with each other to manage the user data (D2), the vehicle data (D1), and the determination result data (D5) in association with each other, and
the determination and analysis device (50A, 50B) transmits at least the determination result data (D5) to a medical institution terminal (70) of a medical institution, receives medical information data (D6) created based on the determination result data (D5) and an opinion of a medical worker at the medical institution from the medical institution terminal (70), and notifies the user terminal (60, 60A, 60B) of the medical information including a content based on the medical information data (D6).

2. The abnormality determination system according to claim 1, wherein
the medical information data includes information related to at least one of a treatment program determined to be suitable for the user by the medical worker, a determination result related to a cognitive function of the user, and a consultation recommendation on a disease related to the cognitive function.

3. The abnormality determination system according to claim 2, wherein
in a case where the medical information data includes the information related to the consultation recommendation,
the determination and analysis device transmits the medical information including presentation of a behavior recommended to the user to the user terminal, and
the user terminal presents the behavior recommended to the user and displays an operation of executing the behavior in a receivable manner.

4. The abnormality determination system according to any one of claims 1 to 3, wherein
the traveling information includes information related to a driving operation of the vehicle, and
the determination and analysis device determines the abnormal indication of the user based on determination reference data including a correlation relation between the driving operation of the vehicle and a cognitive function state of the user

5. The abnormality determination system according to any one of claims 1 to 3, wherein
the mobility server and the medical server manage the user data, the vehicle data, and the determination result data in association with each other for each user, and
the user data includes information that is collatable with diagnosis information of a patient stored at the medical institution.

6. The abnormality determination system according to any one of claims 1 to 3, wherein
the vehicle data includes vehicle identification information for identifying the vehicle,
the mobility server further manages the vehicle data and stores the user data and the vehicle identification information in association with each other,
the medical server stores the determination result data and the vehicle identification information in association with each other, and
the determination and analysis device transmits, to the medical institution terminal, the user data and the determination result data in association with each other based on the vehicle identification information.

7. The abnormality determination system according to claim 5, wherein
the medical server acquires, from the vehicle data stored in the mobility server, only the vehicle data utilized by the determination and analysis device to perform determination related to the abnormal indication of the user

8. A base station device (30), comprising:
a mobility server (40A) configured to store user data (D2) related to a user of a vehicle (V);
a medical server (40B) configured to store vehicle data (D1) including traveling information (D13) of the vehicle (V); and
a determination and analysis device (50A, 50B) configured to determine an abnormal indication of the user from the vehicle data (D1), store determination result data (D5) including a determination result in the medical server (40B), and issue a notification of medical information to the user based on the determination result data (D5), wherein
the mobility server (40A) and the medical server (40B) cooperate with each other to manage the user data (D2), the vehicle data (D1), and the determination result data (D5) in association with each other, and
the determination and analysis device (50A, 50B) transmits at least the determination result data (D5) to a medical institution terminal (70) of a medical institution, receives medical information data (D6) created based on the determination result data (D5) and an opinion of a medical worker at the medical institution from the medical institution terminal (70), and notifies a user terminal (60, 60A, 60B) of the medical information including a content based on the medical information data (D6).
